# EUROPEAN PATENT APPLICATION

(11) **EP 3 646 850 A1**
(43) Date of publication of application: **06.05.2020**
(21) Application number: 18824019.6
(22) Date of filing: 12.06.2018
(51) Int. Cl.: A61K 8/895, A61K 8/34, A61K 8/89, A61Q 1/00, A61Q 1/02, A61Q 17/04

(54) **FILM-FORMING AGENT FOR COSMETICS AND COSMETICS CONTAINING SAME**

(30) Priority: 28.06.2017 JP 2017126794
(71) Applicant: Dow Toray Co., Ltd., Shinagawa-ku Tokyo 1408617 (JP)
(72) Inventor: SOUDA Tatsuo, Ichihara-shi Chiba 299-0108 (JP); KIKUNAGA Sayuri, Ichihara-shi Chiba 299-0108 (JP); HORI Seiji, Ichihara-shi Chiba 299-0108 (JP)
(74) Representative: Thom, Russell
(86) International application number: PCT/JP2018/022413
(87) International publication number: WO 2019/003898

(57) **Abstract**

[Problem] To provide a film-forming agent for cosmetics, which can form a film having excellent water resistance and excellent sebum resistance while also having high washability, and a cosmetic containing this film-forming agent for cosmetics.

[Solution] A film-forming agent for cosmetics, which contains a copolymer polymerized from a monomer composition containing (A) an unsaturated monomer having at least one polysiloxane structure in the molecule and (B) an unsaturated monomer having at least one acidic group or salt thereof in the molecule, wherein the content of the monomer (A) is at least 30 wt% with reference to the monomer composition.

## Description

### Technical Field

The present invention relates to a film-forming agent for cosmetics and to a cosmetic containing the same, and more specifically to a film-forming agent for cosmetics containing a copolymer having a polysiloxane structure and an acidic group which provides a cosmetic having excellent water resistance and excellent sebum resistance while also having high washability, and to a cosmetic containing the same.

### Background Art

Attempts have been made to improve the water resistance and sebum resistance of cosmetics in order to provide longer-lasting cosmetics, especially makeup. The use of polymers containing organopolysiloxanes to improve the water resistance of cosmetics is well known (see, for example, Patent Document 1). While cosmetics whose water resistance and sebum resistance have been improved by the use of these film-forming agents are longer lasting, they can be difficult to wash off when the cosmetics are removed. As a result, the burden is increased on the user who must repeatedly wash off the cosmetic using a special cleansing material or an oil-based makeup remover and a water-based cleanser.

Surface-treated powders coated with a hydrophobizing agent or a polymer having a hydrophilic group are known to be used in cosmetics in order to improve the washability of cosmetics (Patent Document 2). However, this makes the production process more complicated because a powder such as titanium oxide has to be surface treated before being added to the cosmetic.

### Prior Art Documents

### Patent Documents

Patent Document 1: JP 2000-063225 A
Patent Document 2: JP 2007-277167 A

### Summary of the Invention

### Problem to be Solved by the Invention

Therefore, it is an object of the present invention to provide a film-forming agent for cosmetics which can form a film having excellent water resistance and excellent sebum resistance while also having high washability, and to provide a cosmetic containing this film-forming agent for cosmetics.

### Means for Solving the Problem

As a result of extensive research, the present inventors discovered that a film having the contradictory properties of high water resistance and excellent washability could be formed by a composition comprising a copolymer obtained by polymerizing a monomer having a polysiloxane structure and a monomer having an acidic group. The present invention is a product of this discovery.

One aspect of the present invention is a film-forming agent for a cosmetic comprising a copolymer polymerized from a monomer composition containing (A) an unsaturated monomer having at least one polysiloxane structure in the molecule and (B) an unsaturated monomer having at least one acidic group or salt thereof in the molecule, wherein the content of monomer (A) in the monomer composition is at least 30 wt%. The acid value of the copolymer is from 5 to 300 mgKOH/g. The ratio of the weight of monomer (A) to the weight of monomer (B) (A/B) is preferably from 1.0 to 20.0.

Preferably, the unsaturated monomer having at least one polysiloxane structure in the molecule is selected from one represented by General Formula (1)

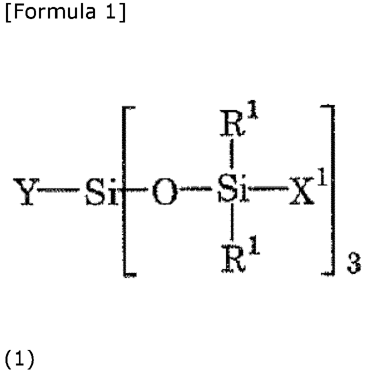

{In this formula, Y is a radically polymerizable organic group, R¹ is an alkyl or aryl group having from 1 to 10 carbon atoms, and X¹ is a silylalkyl group represented by the following formula where i = 1.

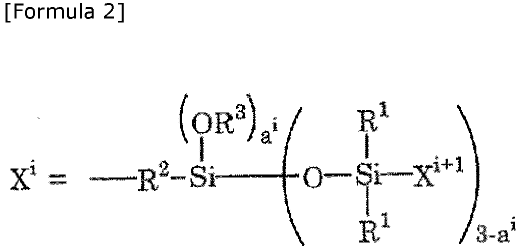

(In this formula, R¹ is the same as above, R² is an alkylene group having from 2 to 10 carbon atoms, R³ is an alkyl group having from 1 to 10 carbon atoms, Xⁱ⁺¹ is a hydrogen atom or a group selected from the group consisting of an alkyl group having from 1 to 10 carbon atoms, an aryl group, and a silylalkyl group mentioned above, i is an integer from 1 to 10 representing the number of levels of silylalkyl groups mentioned above, and aⁱ is an integer from 0 to 3.)} or one represented by General Formula (2).

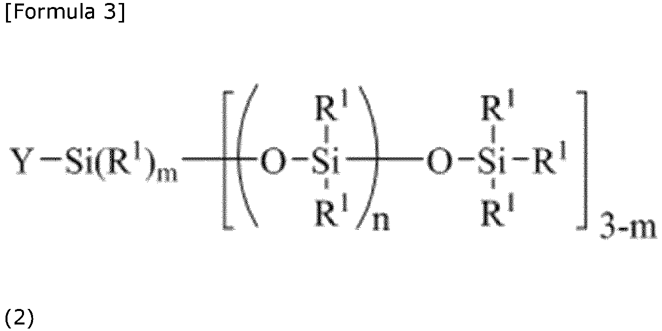

(In this formula, Y and R¹ are the same as above, m is 0, 1 or 2, and n is a number from 0 to 200 representing the average degree of polymerization.)

The monomer composition may further comprise (C) a monomer having at least one carboxylic acid ester in the molecule. Also, monomer (B) is preferably acrylic acid.

A film-forming agent for a cosmetic in the present invention may further comprise at least one selected from the group consisting of (D) an oil, (E) an alcohol and (F) a surfactant, and may further comprise at least one selected from the group consisting of water, an inorganic powder, an organic powder, a colorant, a thickener, a gelling agent, an organically modified clay mineral, a silicone resin, a silicone gum, a silicone elastomer, an organically modified silicone, a UV protection component, a water-soluble polymer, an organic resin, a moisturizer, a preservative, an antioxidant, an antibacterial agent, a fragrance, a salt, a pH regulator, a chelating agents, an algefacient, an anti-inflammatory, a skin-beautifying component, a vitamin, an amino acid, a nucleic acid, a hormone, an inclusion compound, and an antistatic agent.

A second aspect of the present invention is a cosmetic comprising a film-forming agent for a cosmetic described above.

### Effects of the Invention

The present invention is able to provide a film-forming agent for cosmetics which can form a film having excellent water resistance and excellent sebum resistance while also having high washability, and to provide a cosmetic containing this film-forming agent for cosmetics.

### Brief Description of the Drawings

[FIG. 1]

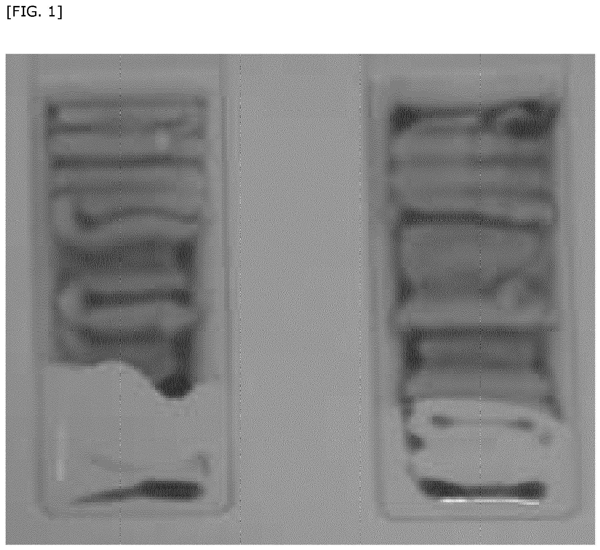

FIG. 1 is a photograph showing the results of Washability Test 2 in which the lower portion of the glass plate has been washed.

### Embodiment of the Invention

In the present specification, "(meth) acrylic acid" refers to both acrylic acid and methacrylic acid. Similarly, "(meth) acrylate", "(meth) acryloxy", and "(meth) acrylamide" refer, respectively, to acrylate and methacrylate, acryloxy and methacryloxy, and acrylamide and methacrylamide. In the present invention, "cosmetic" and "cosmetic product" are used interchangeably.

In the present invention, "film-forming agent" refers to a composition containing components used for the purpose of forming a polymer film that adheres to a substrate and conforms to a substrate when applied to a substrate, and is applied especially to keratin in the hair and skin. In the present invention, this refers to a composition for forming a film of a copolymer polymerized from a monomer composition containing monomer (A) and monomer (B) on a substrate (a "film-forming agent for cosmetics" below) and to the use of this composition. Forming this film on skin can keep makeup from coming off, prevent discoloration, prevent the uneven distribution of active ingredients such as UV absorbers, and make a cosmetic last longer. Most film-forming agents are polymer compounds. Silicone resins, fluorine resins and acrylic resins are used most often, and these are most commonly blended into sunscreen lotions, makeup, and lipstick.

In the present invention, the film-forming agent for a cosmetic comprises a copolymer polymerized from a monomer composition containing an unsaturated monomer having at least one polysiloxane structure in the molecule (component (A)) and an unsaturated monomer having at least one acidic group or salt thereof in the molecule (component (B)). The unsaturated monomer having a polysiloxane structure in component (A) is used to introduce a polysiloxane structure to the copolymer. This unsaturated monomer is preferably selected from compounds represented by General Formula (1) or General Formula (2) below.

### General Formula (1)

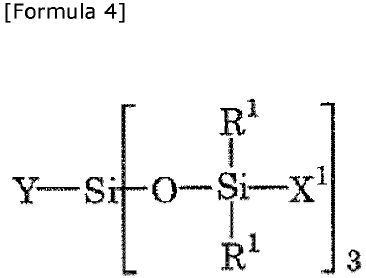

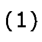

In General Formula (1), Y is an unsaturated group. Specific examples include (meth) acryloxy group-containing organic groups represented by the general formulas below, (meth) acrylamide group-containing organic groups, styryl group-containing organic groups, or alkenyl groups having from 2 to 10 carbon atoms.

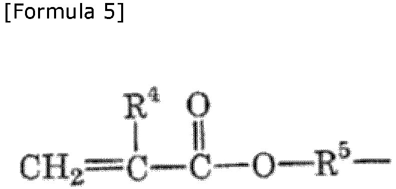

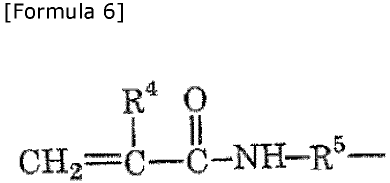

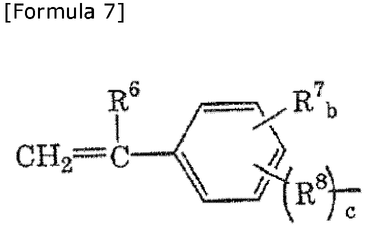

(In these formulas, R⁴ and R⁶ are a hydrogen atom or methyl group, R⁵ and R⁸ are an alkylene group having from 1 to 10 carbon atoms, R⁷ is an alkyl group having from 1 to 10 carbon atoms, b is an integer from 0 to 4, and c is 0 or 1.) Examples of these radically polymerizable organic groups include an acryloxymethyl group, 3-acryloxypropyl group, methacryloxymethyl group, 3-methacryloxypropyl group, 4-vinylphenyl group, 3-vinylphenyl group, 4-(2-propenyl) phenyl group, 3-(2-propenyl) phenyl group, 2-(4-vinylphenyl) ethyl group, 2-(3-vinylphenyl) ethyl group, vinyl group, allyl group, methallyl group, and 5-hexenyl group. R¹ is an alkyl group or aryl group having from 1 to 10 carbon atoms. The alkyl group can be a methyl group, ethyl group, propyl group, butyl group, pentyl group, isopropyl group, isobutyl group, cyclopentyl group, or cyclohexyl group. The aryl group can be a phenyl group or a naphthyl group. Among these, a methyl group or phenyl group is preferred and a methyl group is especially preferred. X¹ is a silylalkyl group represented by the following formula where i = 1.

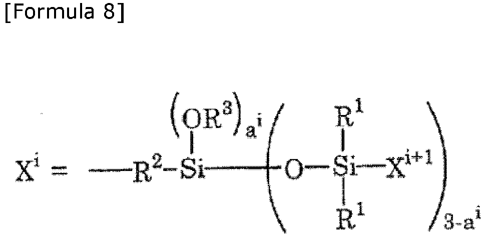

In this formula, R² is an alkylene group having from 2 to 10 carbon atoms. Examples include linear alkylene groups such as an ethylene group, propylene group, butylene group and hexylene group; and branched alkylene groups such as a methylmethylene group, methylethylene group, 1-methylpentylene group and 1,4-dimethylbutylene group. Among these, an ethylene group, methylethylene group, hexylene group, 1-methylpentylene group or 1,4-dimethylbutylene group is preferred. R³ is an alkyl group having from 1 to 10 carbon atoms. Examples include a methyl group, ethyl group, propyl group, butyl group and isopropyl group. R¹ is the same as above. Xⁱ⁺¹ is a hydrogen atom or a group selected from the group consisting of an alkyl group having from 1 to 10 carbon atoms, an aryl group, and a silylalkyl group mentioned above. aⁱ is an integer from 0 to 3, preferably from 0 to 2, more preferably 0 to 1, and even more preferably 0. i is an integer from 1 to 10 and represents the number of levels of silylalkyl groups mentioned above, that is, the number of repeating silylalkyl groups. Therefore, when the number of levels is 1, the carbosiloxane dendrimer in this component is represented by the following general formula.

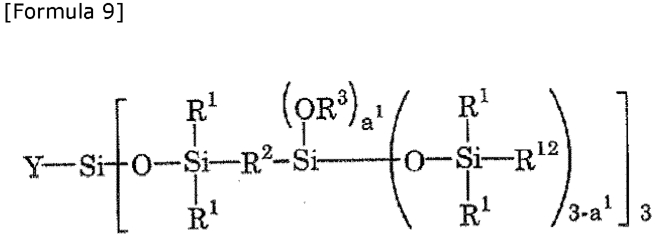

(In this formula, Y, R¹, R² and R³ are the same as above, R¹² is a hydrogen atom or the same as R¹ above, and a¹ is the same as aⁱ above except that the average total number of a¹ per molecule is from 0 to 7.) When the number of levels is 2, the carbosiloxane dendrimer in this component is represented by the following general formula.

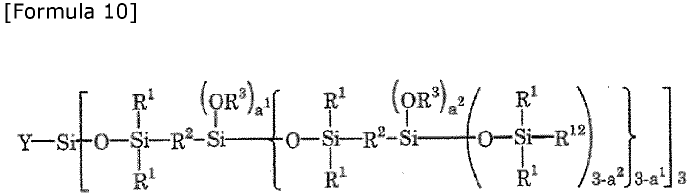

(In this formula, Y, R¹, R², R³ and R¹² are the same as above, and a¹ and a² are the same as aⁱ above except that the average total number of a¹ and a² per molecule is from 0 to 25.) When the number of levels is 3, the carbosiloxane dendrimer in this component is represented by the following general formula.

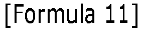

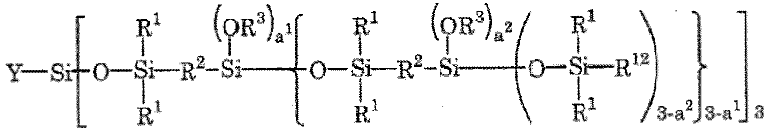

(In this formula, Y, R¹, R², R³ and R¹² are the same as above, and a¹, a² and a³ are the same as aⁱ above except that the average total number of a¹, a² and a³ per molecule is from 0 to 79.)

Examples of carbosiloxane dendrimers containing radically polymerizable organic groups in this component include the carbosiloxane dendrimers represented by the following average composition formulas.

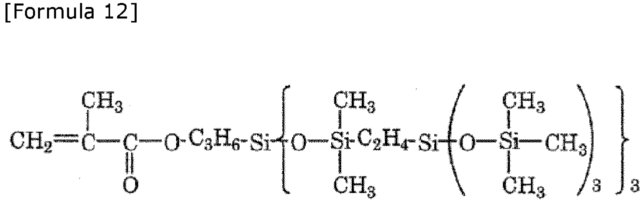

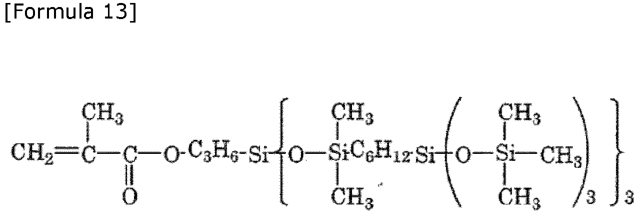

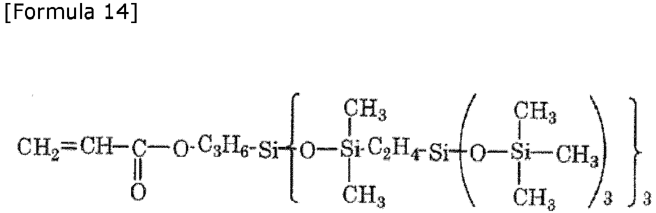

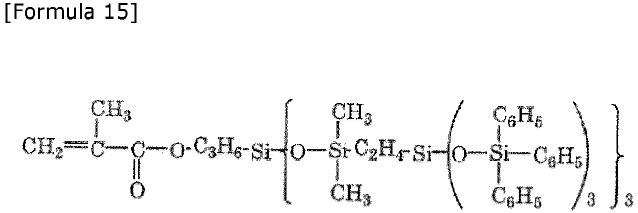

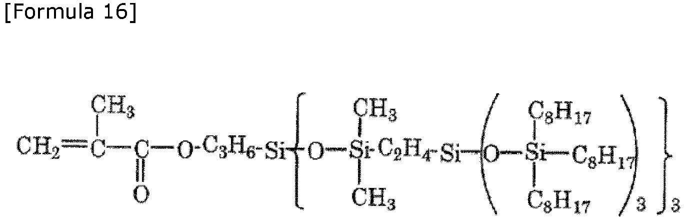

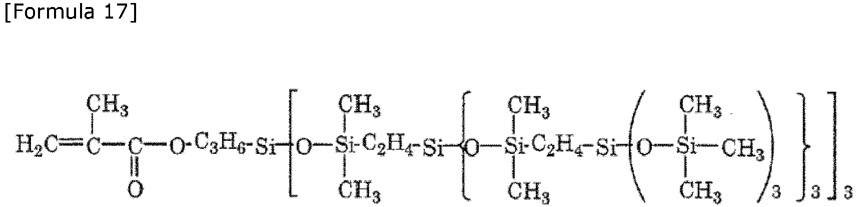

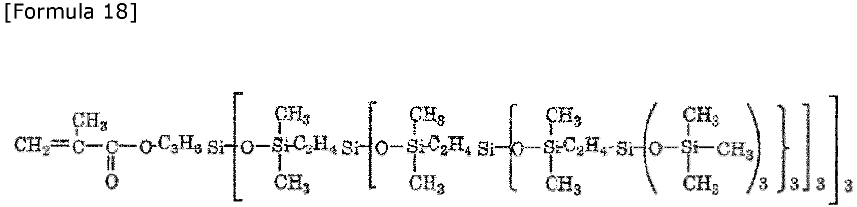

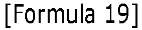

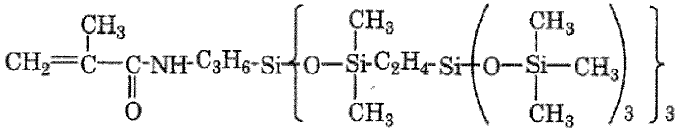

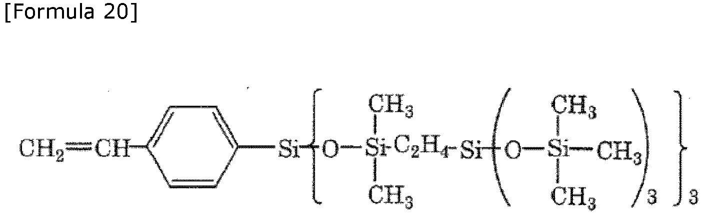

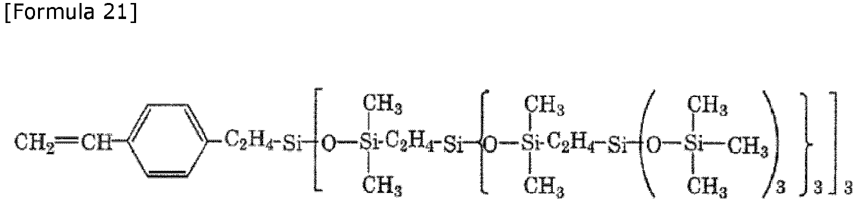

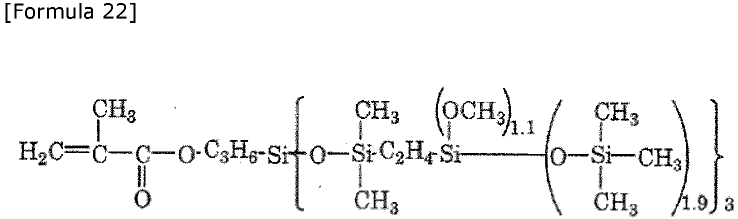

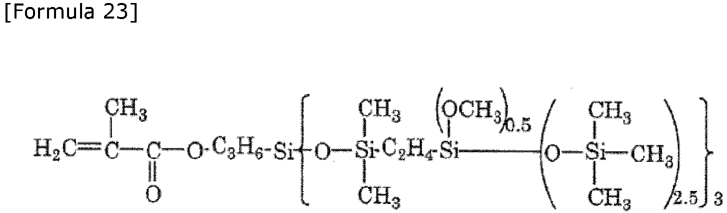

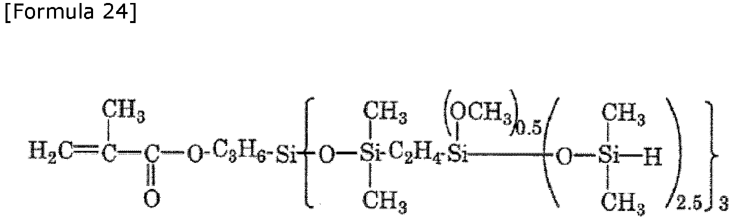

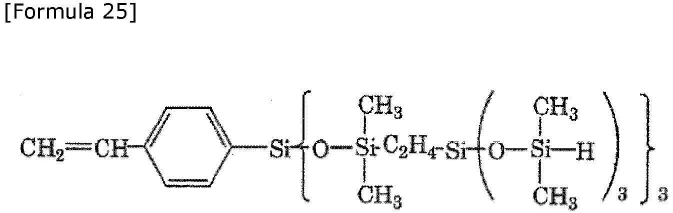

These carbosiloxane dendrimers can be produced using the production method for branched siloxane/silalkylene copolymers described in JP H11-001530 A (Appl. No. H09-171154). For example, one can be produced by conducting a hydrosilylation reaction on a silicon compound containing a silicon atom-bonded hydrogen atom represented by the following general formula 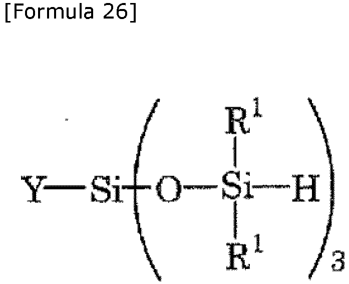 (where R¹ and Y are the same as above) and an alkenyl group-containing organosilicon compound. Examples of these silicon compounds that can be used include 3-methacryloxypropyltris (dimethylsiloxy) silane, 3-acryloxypropyltris (dimethylsiloxy) silane, and 4-vinylphenyltris (dimethylsiloxy) silane. Examples of these alkenyl group-containing organosilicon compounds that can be used include vinyl tris (trimethylsiloxy) silane, vinyl tris (dimethylphenylsiloxy) silane, and 5-hexenyltris (trimethylsiloxy) silane. The hydrosilylation reaction is preferably conducted in the presence of a transition metal catalyst such as chloroplatinic acid or a platinum vinyl siloxane complex.

Another preferred embodiment of a compound used in the present invention that is an unsaturated monomer having a polysiloxane structure is represented by General Formula (2) below.

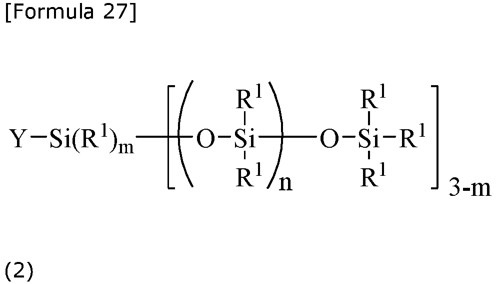

(In this formula, Y and R¹ are the same as above, m is 0, 1 or 2, and n is a number from 0 to 200 representing the average degree of polymerization.)

Specific examples of monomers represented by General Formula (2) include the following compounds.

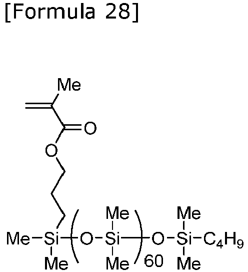

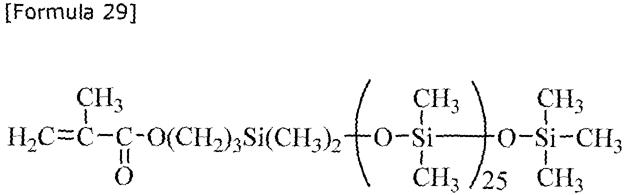

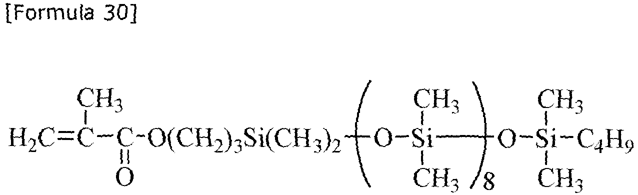

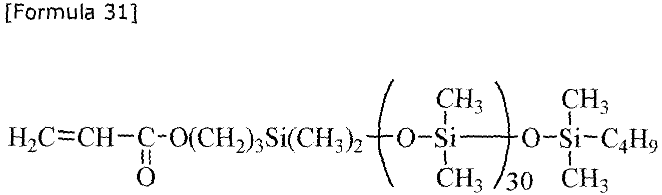

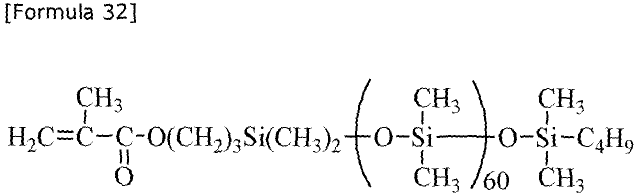

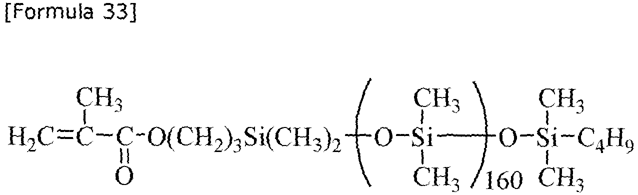

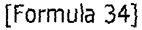

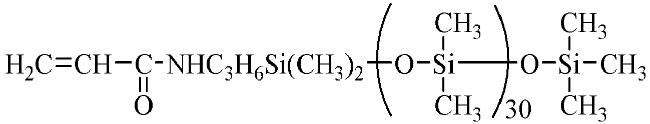

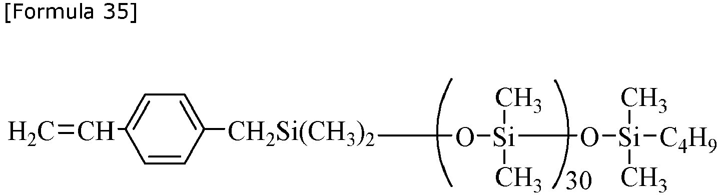

The following is an example of a compound in which m is 0 and n is 0 in General Formula (2). It can be used as one embodiment of monomer (A) in the present invention.

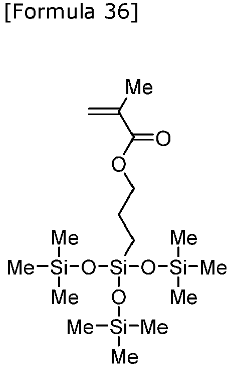

The content of monomer (A) is 30% or more, and preferably 40% or more, in terms of weight in the monomer composition. When at least this amount of monomer (A) is used in terms of weight, the water repellency and oil repellency of the resulting copolymer are higher and the water resistance and sebum resistance of a cosmetic using this copolymer are improved. Also, the content of monomer (A) is preferably 70% or less, and more preferably 60% or less, in terms of weight in the monomer composition.

### Unsaturated monomer having at least one acidic group or salt thereof in the molecule (Component (B)):

The unsaturated monomer having at least one acidic group or salt thereof in the molecule in component (B) of the present invention is a compound having a radically polymerizable vinyl group and at least one acidic group or salt thereof in the molecule. Examples of acidic groups include carboxylic acids, sulfonic acids and phosphonic acids. Examples of salts thereof include alkali metal salts, alkaline earth metal salts, basic amino acid salts, ammonium salts, alkyl ammonium salts, alkylamine salts, and alkanolamine salts. Specific examples include sodium salts, potassium salts, magnesium salts, calcium salts, L-arginine salts, L-histidine salts, L-lysine salts, ammonium salts, triethanolamine salts, aminomethylpropanediol salts, and mixtures of these salts. The hydrophilicity-hydrophobicity of compounds having these acidic groups is changed in an aqueous solution by the release of a proton (H⁺) at a specific pH or by binding with a cation component in the solution to form a salt. Similarly, the hydrophilicity-hydrophobicity of compounds having salts of these acidic groups is changed by salt dissociation occurring at a specific pH. Therefore, blending the right compound having these acidic groups or salts thereof into a cosmetic has the effect of producing a long-lasting makeup that is also easy to wash off when removed.

Examples of unsaturated monomers having at least one acidic group or salt thereof in the molecule include (meth) acrylic acid, crotonic acid, maleic acid, fumaric acid, itaconic acid, angelic acid, tigulinic acid, 2-carboxyethyl acrylate oligomers, styrene sulfonic acid, mono-[(2-hydroxyethyl) methacrylic acid] phosphate ester, mono-[(2-hydroxyethyl) acrylic acid] phosphate ester, ((2-hydroxyethyl) methacrylic acid) diphosphate ester, di [(2-hydroxyethyl) acrylic acid] phosphate ester, and salts thereof. In order to realize good washability without adversely affecting the water resistance of the resulting copolymer, the amount of monomer (B) has to be equal to or less than the amount of monomer (A), and is preferably a small amount. More specifically, the ratio of the weight of monomer (A) to the weight of monomer (B) (A/B) is in a range from 1.0 to 20.0, preferably in a range from 2 to 15, and more preferably in a range from 2 to 12. From the standpoint of realizing the technical effects of the present invention, component (B) is preferably (meth) acrylic acid and salts thereof. From the standpoint of realizing both water resistance and washability, acrylic acid and salts thereof are especially preferred. The unsaturated monomers can be commercially available products that are used directly or after being purified. When copolymers containing monomers derived from acrylic acid and methacrylic acid were compared, the copolymers using monomers derived from acrylic acid tended to have a lower Tg and better film flexibility.

### Other monomers (Component (C)):

In addition to component (A) and component (B) described above, the present invention can contain another monomer as component (C). Component (C) should be copolymerizable with component (A) and component (B). Examples include lower alkyl (meth) acrylates such as (meth) methyl acrylate, (meth) ethyl acrylate, (meth) n-propyl acrylate, and isopropyl (meth) acrylate; glycidyl (meth) acrylate; higher (meth) acrylates such as n-butyl (meth) acrylate, (meth) isobutyl acrylate, tert-butyl (meth) acrylate, n-hexyl (meth) acrylate, cyclohexyl (meth) acrylate, 2-ethylhexyl (meth) acrylate, octyl (meth) acrylate, (meth) lauryl acrylate, stearyl (meth) acrylate, (meth) isostearyl acrylate, and behenyl (meth) acrylate; lower fatty acid vinyl esters such as vinyl acetate and vinyl propionate; higher fatty acid esters such as vinyl butyrate, vinyl caproate, vinyl 2-ethylhexanoate, vinyl laurate, and vinyl stearate; vinyl aromatic monomers such as styrene, vinyl toluene, benzyl (meth) acrylate, phenoxyethyl (meth) acrylate, and vinyl pyrrolidone; amide group-containing vinyl monomers such as (meth) acrylamide, N-methylol (meth) acrylamide, N-methoxymethyl (meth) acrylamide, isobutoxymethoxy (meth) acrylamide, and N,N-dimethyl (meth) acrylamide; hydroxyl-containing vinyl monomers such as hydroxyethyl (meth) acrylate and hydroxypropyl alcohol (meth) acrylate; ether bond-containing vinyl monomers such as tetrahydrofurfuryl (meth) acrylate, butoxyethyl (meth) acrylate, ethoxydiethylene glycol (meth) acrylate, polyethylene glycol (meth) acrylate, polypropylene glycol mono (meth) acrylate, hydroxybutyl vinyl ether, cetyl vinyl ether, and 2-ethylhexyl vinyl ether; unsaturated group-containing silicone compounds such as (meth) acryloxypropyl trimethoxysilane, polydimethylsiloxane containing a (meth) acrylic group at one end, both ends and/or in a side chain, and polydimethylsiloxane containing a styryl group at one end; butadiene; vinyl chloride; vinylidene chloride; (meth) acrylonitrile; dibutyl fumarate; maleic anhydride; dodecyl succinic anhydride; (meth) acrylic glycidyl ether; quaternary ammonium salts derived from (meth) acrylic acid, such as 2-hydroxy-3-methacryloxypropyl trimethylammonium chloride, methacrylic acid esters of alcohols having a tertiary amine group such as methacrylic acid diethylamine ester, and quaternary ammonium salts of these.

Polyfunctional vinyl monomers can also be used. Examples include trimethylolpropane tri (meth) acrylate, pentaerythritol tri (meth) acrylate, ethylene glycol di (meth) acrylate, tetraethylene glycol di (meth) acrylate, polyethylene glycol di (meth) acrylate, 1,4-butanediol di (meth) acrylate, 1,6-hexanediol di (meth) acrylate, neopentyl glycol di (meth) acrylate, trimethylolpropanetrioxyethyl (meth) acrylate, tris (2-hydroxyethyl) isocyanurate di (meth) acrylate, tris (2-hydroxyethyl) isocyanurate tri (meth) acrylate, and unsaturated group-containing silicone compounds such as styryl-capped polydimethylsiloxane.

As mentioned above, the ratio of the weight of monomer (A) to the weight of monomer (B) (A/B) in the monomer composition is preferably in a range from 1.0 to 20.0, more preferably in a range from 2.0 to 15.0, and even more preferably in a range from 2.0 to 12.0. When monomer (A) and monomer (B) are copolymerized at this weight ratio, the hydrophobic-hydrophilic balance of the resulting copolymer is preferred. When the A/B ratio is less than 2, the washability of the film formed by the composition as a cosmetic material is insufficient.

The total content of monomer (A) and monomer (B) relative to the entire monomer composition ([(A)+(B)]/[(A)+(B)+(C)]) is preferably at least 40% by weight, more preferably at least 50% by weight, and even more preferably at least 55% by weight. When the total content of monomer (A) and monomer (B) relative to the entire monomer composition is within the range mentioned above, a sufficient amount of hydrophobic groups and hydrophilic groups have been introduced to the resulting copolymer, and a cosmetic can be obtained with high water resistance, sebum resistance, and washability.

The method used to copolymerize the copolymer in the film-forming agent can be the radical polymerization method or the ionic polymerization method, but the radical polymerization method is preferred. Solution polymerization is preferably used as the radical polymerization method. In solution polymerization, a monomer composition containing component (A), component (B), and optionally component (C) is reacted in the presence of a radical initiator in a solvent at a temperature from 50 to 150°C for 3 to 20 hours. Examples of solvents that can be used in the polymerization reaction include aliphatic hydrocarbons such as hexane, octane, decane and cyclohexane; aromatic hydrocarbons such as benzene, toluene, and xylene; ethers such as diethyl ether, dibutyl ether, tetrahydrofuran, and dioxane; ketones such as acetone, methyl ethyl ketone, methyl isobutyl ketone, and diisobutyl ketone; esters such as methyl acetate, ethyl acetate, butyl acetate, and isobutyl acetate; alcohols such as methanol, ethanol, isopropyl alcohol, and butanol; and organosiloxane oligomers such as octamethylcyclotetrasiloxane, decamethylcyclopentasiloxane, hexamethyldisiloxane, and octamethyltrisiloxane.

Any radical initiator commonly used in the radical polymerization method can be used. Specific examples include azobis compounds such as 2,2'-azobis (isobutyronitrile), 2,2'-azobis (2-methylbutyronitrile), and 2,2'-azobis (2,4-dimethylvaleronitrile); and organic peroxides such as benzoyl peroxide, lauroyl peroxide, tert-butyl peroxybenzoate, tert-butylperoxy-2-ethylhexanoate, and tert-hexylperoxy-2-ethylhexanoate. These radical initiators can be used alone or on mixtures of two or more. The amount of radical initiator used is preferably in a range from 0.1 to 5 parts by weight per 100 parts by weight monomer composition.

A chain transfer agent can also be added during polymerization. Specific examples of chain transfer agents include mercapto compounds such as 2-mercaptoethanol, butyl mercaptan, n-dodecyl mercaptan, 3-mercaptopropyltrimethoxysilane, and polydimethylsiloxanes having a mercaptopropyl group; and halides such as methylene chloride, chloroform, carbon tetrachloride, butyl bromide, and 3-chloropropyltrimethoxysilane.

The polymerization reaction product resulting from this process is then brought into contact with a palladium catalyst. By bringing the reaction product into contact with a palladium catalyst, the vinyl groups in the unreacted monomer remaining in the polymerization reaction product are saturated, and the irritation and odor of the product can be reduced before being added to cosmetics. Examples of palladium catalysts include, but are not limited to, palladium compounds such as tetrakis (triphenylphosphine) palladium (0) and dichlorobis (triphenylphosphine) palladium (II), as well as carbon-supported palladium, carbon-supported palladium hydroxide and platinum oxide. Carbon-supported palladium is the preferred catalyst. The catalyst can be another metal such as nickel, but the polymerization product contains an acidic group and a nickel catalyst gradually elutes into the reaction system under acidic conditions. While palladium is a precious metal, this particular problem does not occur when a carbon-supported palladium catalyst is used as a heterogeneous catalyst. As a result, it is preferably used as the catalyst in the present invention.

The temperature at which the polymerization reaction product is brought into contact with the palladium catalyst is from 50 to 200°C, and preferably from 70 to 130°C. The pressure (absolute pressure) is from 1 to 1,000 kg/cm², and preferably from 2 to 100 kg/cm². The contact time is from 1 to 15 hours, and preferably 3 to 10 hours. The reaction can be performed in a solvent, and the solvent may be used directly during polymerization or solvent replacement may be performed. The solvent can be one of those mentioned above in relation to the polymerization reaction.

Stripping, reprecipitation, and filtration may also be performed during this process. Stripping, reprecipitation, filtration, pulverization, and/or classification can be performed after contact with the palladium catalyst.

The presence or absence of unreacted monomers in the resulting copolymer can be confirmed by the presence of a peak integrated value for ethylenically unsaturated groups (5.5 to 6.5 ppm) in ¹H-NMR. The end of the reaction can be confirmed by the disappearance or reduction in the peak derived from ethylenically unsaturated groups. More specifically, the comparison can be made using the ratio of the peak integrated value for ethylenically unsaturated groups to the product of the integrated value (0 to 0.3 ppm) for methyl groups derived from the unsaturated monomer having the polysiloxane structure and the weight percentage of the unsaturated monomer having the polysiloxane structure when added to the system (the residual unsaturation ratio). The residual unsaturation ratio for the copolymer is 0.1 or less, and preferably 0.02 or less.

After polymerization, purification can be performed by reducing the pressure under heating to remove the remaining unreacted vinyl monomers, by performing hydrogenation in the presence of a hydrogenation catalyst and in the presence or absence of a solvent to deodorize the product, and/or by contacting the product with nitrogen gas under reduced pressure to remove light substances. A purified product is especially preferred when used in external preparations which require low odor and compatibility with other cosmetic components. There are no particular restrictions on the solvents, reaction conditions, and reduced pressure conditions used in the hydrogenation reaction stripping process. Any solvent, reaction conditions, and reduced pressure conditions commonly used to purify organopolysiloxane copolymers can be selected.

The acid value of a copolymer of the present invention as determined according to JIS 1557-5 is preferably from 5 to 300 mgKOH/g, and more preferably from 35 to 100 mgKOH/ g. When the acidic group in a copolymer of the present invention is in the form of a salt, the acid value after the salt has be subjected to an exchange with hydrogen ions to form an acid is used. The number average molecular weight of the copolymer is preferably from 1,000 to 200,000, and more preferably from 2,000 to 80,000, from the standpoint of easier incorporation into cosmetics. It can be in the form of a liquid, gum, paste, solid, or powder.

A film-forming agent for cosmetics in the present invention can be blended into a cosmetic directly or in the form of a composition dissolved in a solvent or dispersed in a dispersion medium.

In addition to the copolymer described above, a film-forming agent for cosmetics in the present invention may contain at least one selected from the group consisting of (D) an oil and (E) an alcohol. When blended into a cosmetic containing the copolymer, these can take the form of a solution previously dissolved in a solvent, a dispersion liquid previously dispersed in a dispersion medium, or a solid such as a powder, granules, or a block. A film-forming agent for cosmetics in the present invention is preferably blended into a cosmetic in the form of a copolymer composition containing the copolymer and an oil or alcohol obtained by dissolving or dispersing the copolymer in one or more oil or alcohol.

A film-forming agent for cosmetics in the present invention has exceptional compatibility with and dispersibility in various types of oils or alcohols, and can be used to obtain a composition that remains uniform over a long period of time. This composition can be blended directly into cosmetics, and is an extremely useful ingredient in cosmetics from the standpoint of handling and storage stability. A composition containing from 5 to 1,000 parts by mass, preferably from 50 to 500 parts by mass, and more preferably from 100 to 400 parts by mass of at least one selected from a group consisting of oils and alcohols per 100 parts by mass copolymer of the present invention is especially useful. When a composition contains a copolymer of the present invention and an oil or alcohol, the solvent and unreacted monomer may be removed after polymerization, and the copolymer uniformly dispersed in the oil or alcohol using mechanical force. Alternatively, the volatile solvent may be replaced by the oil or alcohol during polymerization. There are no particular restrictions on the amount blended into cosmetics, but a copolymer of the present invention can be blended into a cosmetic in a range from 0.1 to 50 mass% of the entire cosmetic, preferably from 1 to 10 mass%. When the amount added is within this range, the properties of a copolymer of the present invention can be imparted to a cosmetic, namely, film forming properties and film washability.

### (D) Oil

The oil can be any animal oil, vegetable oil, or mineral oil commonly used in cosmetics. The oil can be solid, semi-solid or liquid and can be non-volatile, semi-volatile or volatile. An oil is used to impart lubrication to skin and hair, and to make skin soft and impart a moist feeling. An oil can also be used to dilute a copolymer of the present invention to obtain a copolymer composition. The oil is preferably at least one type selected from among (D1) a silicone oil and (D2) an organic oil that is a liquid at a temperature from 5 to 100°C. The type and viscosity of the oil depends on the type of cosmetic and the intended use. These oils are blended into a cosmetic of the present invention at the same time as the composition.

### (D1) Silicone Oils

The silicone-based oils are generally hydrophobic and their molecular structures may be cyclic, linear or branched. Here, the molecular structure may be cyclic, linear or branched. The viscosity of the silicone-based oil at 25°C is usually in a range from 0.65 to 100,000 mm²/s and preferably in a range from 0.65 to 10,000 mm²/s. The silicone oil agent may have volatility and this is preferred.

Examples of silicone-based oils include cyclic organopolysiloxanes, linear organopolysiloxanes and branched organopolysiloxanes. Among these, volatile cyclic organopolysiloxanes, linear organopolysiloxanes and branched organopolysiloxanes are preferred.

The silicone oil can be an organopolysiloxane represented by General Formula (3), (4) or (5) below.

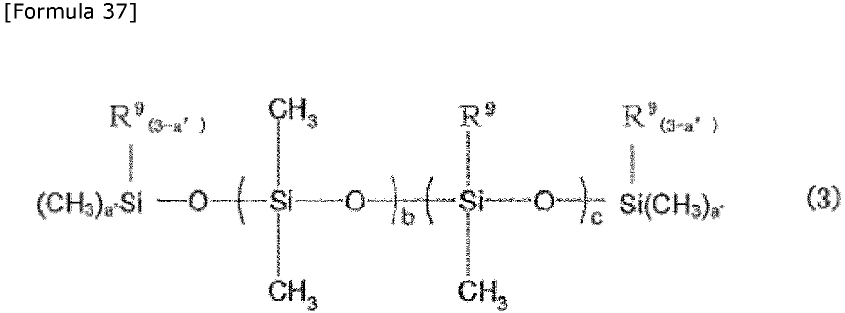

(In this formula, R⁹ is a hydrogen atom or a group selected from among a hydroxyl group, a monovalent unsubstituted or fluorine- or amino-substituted alkyl group, aryl group and alkoxy group having from 1 to 30 carbon atoms, and (CH₃)₃SiO{(CH₃)₂SiO}ISi(CH₃) ₂CH₂CH₂- (where I is an integer from 0 to 1,000), a' is an integer from 0 to 3, b is an integer from 0 to 1,000, and c is an integer from 0 to 1000, provided 1 ≤ b + c ≤ 2,000.)

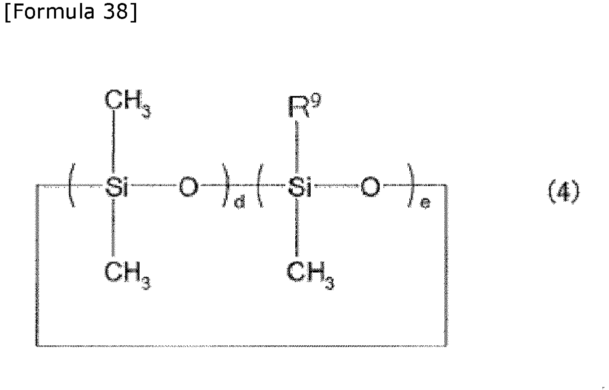

(In this formula, R⁹ is the same as above, d is an integer from 0 to 8, and e is an integer from 0 to 8, provided 3 ≤ d + e ≤ 8.)
[Formula 39]

R⁹₍₄₋₁)Si(OSiCH₃)_{g} (5)

(In this formula, R⁹ is the same as above, f is an integer from 1 to 4, and g is an integer from 0 to 500.)

Examples of monovalent unsubstituted or fluorine- or amino-substituted alkyl groups, aryl groups and alkoxy groups having from 1 to 30 carbon atoms include linear or branched alkyl groups having from 1 to 30 carbon atoms such as a methyl group, an ethyl group, a propyl group, a butyl group, a pentyl group, a hexyl group, a heptyl group, an octyl group, a decyl group, and a dodecyl group; cycloalkyl groups having from 3 to 30 carbon atoms such as a cyclopentyl group and a cyclohexyl group; aryl groups having from 6 to 30 carbon atoms such as a phenyl group, a tolyl group, a xylyl group, and a naphthyl group; alkoxy groups having 1 to 30 carbon atoms such as a methoxy group, an ethoxy group, and a propoxy group; and groups in which hydrogen atoms bonded to carbon atoms in any of these groups have been at least partially replaced by a fluorine atom or an amino group. An unsubstituted alkyl group or aryl group is preferred, an unsubstituted alkyl group or aryl group having from 1 to 6 carbon atoms is more preferred, and a methyl group, ethyl group or phenyl group is especially preferred.

Examples of silicone oils having these structures include cyclic organopolysiloxanes. Specific examples include hexamethylcyclotrisiloxane, octamethylcyclotetrasiloxane, decamethylcyclopentasiloxane, dodecamethylcyclohexasiloxane, 1,1-ethylhexamethylcyclotetrasiloxane, phenylheptamethylcyclotetrasiloxane, 1,1-diphenylhexamethylcyclotetrasiloxane, 1,3,5,7-tetravinyltetramethylcyclotetrasiloxane, 1,3,5,7-tetramethylcyclotetrasiloxane, 1,3,5,7-tetracyclohexyltetramethylcyclotetrasiloxane, tris (3,3,3-trifluoropropyl) trimethylcyclotrisiloxane, 1,3,5,7-tetra (3-methacryloxypropyl) tetramethylcyclotetrasiloxane, 1,3,5,7-tetra (3-acryloxypropyl) tetramethylcyclotetrasiloxane, 1,3,5,7-tetra (3-carboxypropyl) tetramethylcyclotetrasiloxane, 1,3,5,7-tetra (3-vinyloxypropyl) tetramethylcyclotetrasiloxane, 1,3,5,7-tetra (p-vinylphenyl) tetramethylcyclotetrasiloxane, 1,3,5,7-tetra [3- (p-vinylphenyl) propyl] tetramethylcyclotetrasiloxane, 1,3,5,7-tetra (N-acryloyl-N-methyl-3-aminopropyl) tetramethylcyclotetrasiloxane, and 1,3,5,7-tetra (N, N-bis (lauroyl)-3-aminopropyl) tetramethylcyclotetrasiloxane.

Examples of linear organopolysiloxanes include dimethylpolysiloxane capped at both ends of the molecular chain with a trimethylsiloxy group (dimethyl silicone with a low viscosity of 2 mPa·s or 6 mPa·s to dimethyl silicone with a high viscosity of 1 million mPa·s), organohydrogenpolysiloxane, methylphenylpolysiloxane capped at both ends of the molecular chain with a trimethylsiloxy group, dimethylsiloxane/methylphenylsiloxane copolymers capped at both ends of the molecular chain with a trimethylsiloxy group, diphenyl polysiloxane capped at both ends of the molecular chain with a trimethylsiloxy group, dimethylsiloxane/diphenylsiloxane copolymers capped at both ends of the molecular chain with a trimethylsiloxy group, dimethylsiloxane/methylphenylsiloxane copolymers capped at both ends of the molecular chain with a trimethylsiloxy group, diphenyl polysiloxane capped at both ends of the molecular chain with a trimethylsiloxy group, dimethylsiloxane/diphenylsiloxane copolymers capped at both ends of the molecular chain with a trimethylsiloxy group, diphenylpolysiloxane capped at both ends of the molecular chain with a trimethylsiloxy group, dimethylsiloxane/diphenylsiloxane copolymer capped at both ends of the molecular chain with a trimethylsiloxy group, trimethylpentaphenyl trisiloxane, phenyl (trimethylsiloxy) siloxane, methyl alkyl polysiloxane capped at both ends of the molecular chain with a trimethylsiloxy group, dimethylpolysiloxane/methylalkylsiloxane copolymer capped at both ends of the molecular chain with a trimethylsiloxy group, dimethylsiloxane-methyl (3,3,3-trifluoropropyl) siloxane copolymer capped at both ends of the molecular chain with a trimethylsiloxy group, α, ω-dihydroxypolydimethylsiloxane, α, ω-diethoxypolydimethylsiloxane, 1,1,1,3,5,5,5-heptamethyl-3-octyltrisiloxane, 1,1,1,3,5,5,5-heptamethyl-3-dodecyltrisiloxane, 1,1,1,3,5,5,5-heptamethyl-3-hexadecyltrisiloxane, tristrimethylsiloxymethylsilane, tristrimethylsiloxyalkylsilane, tetrakistrimethylsiloxysilane, tetra methyl-1,3-dihydroxydisiloxane, octamethyl-1,7-dihydroxytetrasiloxane, hexamethyl-1,5-diethoxytrisiloxane, hexamethyldisiloxane, octamethyltrisiloxane, higher alkoxy-modified silicones, and higher fatty acid modified silicones.

Examples of branched organopolysiloxanes include methyl tristrimethylsiloxysilane, ethyl tristrimethylsiloxysilane, propyl tristrimethylsiloxysilane, tetrakis trimethylsiloxysilane, and phenyl tristrimethylsiloxysilane.

When a cosmetic or composition of the present invention containing at least one of these silicone-based oils is used as an ingredient in a cosmetic, aging stability can be improved and the smooth feel characteristic of silicone oil can be realized. Among these silicone-based oils, decamethylcyclopentasiloxane a linear organopolysiloxane with a viscosity in the low viscosity range from 2 to 6 mPa·s, 1,1,1,3,5,5,5-heptamethyl-3-octyltrisiloxane (caprylyl methicone), and tris trimethylsiloxymethylsilane (M3T) are especially preferred.

### (D2) Organic Oils

Examples of organic oils include (D2-1) hydrocarbon oils, (D2-2) fatty acid ester oils, higher alcohols, higher fatty acids, oils and fats, and fluorinated oils. There are no particular restrictions in the present invention, but the organic oil is preferably a liquid at a temperature from 5 to 100°C. Also, hydrocarbon oils and/or fatty acid ester oils are preferred. These can be used alone or in combination with other organic oils and/or silicone-based oils. When the appropriate oils are combined, the stability of the composition and/or cosmetic over time is improved, and the feel required of each cosmetic can be imparted. The smooth feel characteristic of silicone oil can be imparted by blending in a silicone-based oil, a refreshing feel can be imparted to the skin by blending in a highly volatile oil, and a smooth feeling and moisturizing effect (moist feeling) can be imparted to the skin and hair by using a hydrocarbon oil and/or fatty acid ester oil in combination with the silicone-based oil.

Examples of hydrocarbon oils (D2-1) include liquid paraffin, light liquid isoparaffin, heavy liquid isoparaffin, vaseline, n-paraffin, isoparaffin, isododecane, isohexadecane, polyisobutylene, hydrogenated polyisobutylene, polybutene, ozokerite, ceresin, microcrystalline wax, paraffin wax, polyethylene wax, polyethylene/polypropylene wax, squalane, squalene, pristane, and polyisoprene. Use of isododecane is especially preferred in a composition of the present invention because it has excellent volatility, excellent compatibility and affinity (combination stability) with other cosmetic ingredients, and imparts a refreshing feel to the skin.

Examples of fatty acid ester oils (D2-2) include hexyldecyl octoate, cetyl octanoate, isopropyl myristate, isopropyl palmitate, butyl stearate, hexyl laurate, myristyl myristate, oleyl oleate, decyl oleate, octyldodecyl myristate, hexyldecyl dimethyloctanoate, cetyl lactate, myristyl lactate, diethyl phthalate, dibutyl phthalate, lanolin acetate, ethylene glycol monostearate, propylene glycol monostearate, propylene glycol dioleate, glyceryl monostearate, glyceryl monooleate, glyceryl tri-2-ethylhexanoate, trimethylolpropane tri-2-ethylhexanoate, ditrimethylolpropane triethylhexanoate, (isostearic acid/sebacic acid) ditrimethylolpropane, trimethylolpropane trioctanoate, trimethylolpropane triisostearate, diisopropyl adipate, diisobutyl adipate, 2-hexyldecyl adipate, di-2-heptylundecyl adipate, diisostearyl malate, hydrogenated castor oil monoisostearate, N-alkyl glycol monoisostearate, octyldodecyl isostearate, isopropyl isostearate, isocetyl isostearate, ethylene glycol di-2-ethylhexanoate, cetyl 2-ethylhexanoate, pentaerythritol tetra-2-ethylhexanoate, octyldodecyl gum ester, ethyl oleate, octyldodecyl oleate, neopentyl glycol dicaprate, triethyl citrate, 2-ethylhexyl succinate, dioctyl succinate, isocetyl stearate, diisopropyl sebacate, di-2-ethylhexyl sebacate, diethyl sebacate, dioctyl sebacate, dibutyl octyl sebacate, cetyl palmitate, octyldodecyl palmitate, octyl palmitate, 2-ethylhexyl palmitate, 2-hexyldecyl palmitate, 2-heptylundecyl palmitate, cholesteryl 12-hydroxystearylate, dipentaerythritol fatty acid ester, 2-hexyldecyl myristate, ethyl laurate, N-lauroyl-L-glutamic acid-2-octyldodecyl ester, di (cholesteryl/behenyl/octyldodecyl) N-lauroyl-L-glutamate, di (cholesteryl/octyldodecyl) N-lauroyl-L-glutamate, di (phytosteryl/behenyl/octyldodecyl) N-lauroyl-L-glutamate, di (phytosteryl/octyldodecyl) N-lauroyl-L-glutamate, isopropyl N-lauroyl sarcosine, diisostearyl malate, neopentyl glycol dioctanoate, isodecyl neopentanoate, isotridecyl neopentanoate, isostearyl neopentanoate, isononyl isononanoate, isotridecyl isononanoate, octyl isononanoate, isotridecyl isononanoate, diethyl pentanediol dineopentanoate, methyl neopentanoate pentanediol, octyldodecyl neodecanoate, 2-butyl-2-ethyl dioctanoate-1, 3-propanediol, pentaerythrityl tetraoctanoate, hydrogenated rosin pentaerythrityl, pentaerythrityl triethylhexanoate, dipentaerythrityl (hydroxystearate/stearate/rosinate), polyglyceryl tetraisostearate, polyglyceryl-10 nonisostearate, polyglyceryl deca (erucate/isostearate/ricinoleate)-8, diglyceryl (hexyldecanoate/sebacate) oligoester, glycol distearate (ethylene glycol distearate), diisopropyl dimer dilinoleate, diisostearyl dimer dilinoleate, dimer (isostearyl/phytosteryl) dilinoleate, dimer (phytosteryl/behenyl) dilinoleate, dimer (phytosteryl/isostearyl/cetyl/stearyl/ behenyl) dilinoleate, dimer dilinoleate dimer dilinoleate, dimer dilinoleyl diisostearate, dimer linoleyl hydrogenated rosin condensate, dimer dilinoleate hydrogenated castor oil, hydroxyalkyl dimer dilinoleyl ether, glyceryl triisooctanoate, glyceryl triisostearate, glyceryl trimyristate, glyceryl triisopalmitate, glyceryl trioctanoate, glyceryl trioleate, glyceryl diisostearate, glyceryl tri (caprylate/caprate), glyceryl tri (caprylate/caprate/myristate/stearate), hydrogenated rosin triglyceride (hydrogenated ester gum), rosin triglyceride (ester gum), glyceryl beiconate eicosanedioate, glyceryl di-2-heptylundecanoate, diglyceryl myristate isostearate, cholesteryl acetate, cholesteryl nonanoate, cholesteryl stearate, cholesteryl isostearate, cholesteryl oleate, cholesteryl 12-hydroxystearate, cholesteryl macadamia oil fatty acid, phytosteryl macadamia nut oil fatty acid, phytosteryl isostearate, cholesteryl soft lanolin fatty acid, cholesteryl hard lanolin fatty acid, cholesteryl long-chain branched fatty acid, cholesteryl long-chain α-hydroxy fatty acid, octyldodecyl ricinoleate, octyldodecyl lanolin fatty acid, octyldodecyl erucate, isostearic acid hydrogenated castor oil, ethyl avocado oil fatty acid, and isopropyl lanolin fatty acid. Lanolin and lanolin derivatives can also be used as fatty acid ester oils.

In addition to ones mentioned above, oils and fats, higher alcohols, higher fatty acids, and fluorine-based oils may be used as an oil, or two or more of these may be used in combination. For example, two or more of the oils listed below may be used in combination. The following are specific examples of additional oils that can be used in the present invention. One or more selected from among these oils and fats, higher alcohols, higher fatty acids, and fluorine-based oils can be used.

Among oils and fats, natural animal and vegetable fats and oils and semi-synthetic fats and oils that can be used include avocado oil, linseed oil, almond oil, Chinese insect wax, eno oil, olive oil, cocoa butter, kapok low, kaya oil, carnauba wax, liver oil, candelilla wax, beef tallow, beef leg fat, beef bone fat, hardened tallow, apricot kernel oil, whale wax, hardened oil, wheat germ oil, sesame oil, rice germ oil, rice bran oil, sugar cane wax, sasanqua oil, safflower oil, shea butter, Chinese tung oil, cinnamon oil, jojoba wax, olive squalane, shellac wax, turtle oil, soybean oil, teaseed oil, camellia oil, evening primrose oil, corn oil, pig lard, rapeseed oil, Japanese tung oil, rice bran wax, germ oil, horse fat, persic oil, palm oil, palm kernel oil, castor oil, hydrogenated castor oil, castor oil fatty acid methyl ester, sunflower oil, grape oil, bayberry wax, jojoba oil, hydrogenated jojoba ester, macadamia nut oil, beeswax, mink oil, cottonseed oil, cotton wax, Japan wax, Japan wax kernel oil, montan wax, coconut oil, hardened coconut oil, tri-coconut oil fatty acid glyceride, sheep fat, peanut oil, lanolin, liquid lanolin, reduced lanolin, lanolin alcohol, hard lanolin, lanolin acetate, lanolin fatty acid isopropyl, POE lanolin alcohol ether, POE lanolin alcohol acetate, lanolin fatty acid polyethylene glycol, POE hydrogenated lanolin alcohol ether, and egg yolk oil. Here, POE refers to polyoxyethylene.

A higher alcohol has from 10 to 30 carbon atoms. A higher alcohol is a saturated or unsaturated monohydric aliphatic alcohol. Some of the hydrocarbon groups may be linear or branched, but linear groups are preferred. Examples of higher alcohols having from 10 to 30 carbon atoms include lauryl alcohol, myristyl alcohol, palmityl alcohol, stearyl alcohol, behenyl alcohol, hexadecyl alcohol, oleyl alcohol, isostearyl alcohol, hexyldecanol, octyldodecanol, cetostearyl alcohol, 2-decyltetradecinol, cholesterol, sitosterol, phytosterol, lanosterol, lanolin alcohol, hydrogenated lanolin alcohol, POE cholesterol ether, monostearyl glycerin ether (batyl alcohol), and monooleyl glyceryl ether (selachyl alcohol). Preferably, in the present invention, a higher alcohol having a melting point of 40 to 80°C is used alone or a combination of higher alcohols having a melting point of 40 to 70°C is used. These higher alcohols, together with a surfactant, form an aggregate called an α-gel, and have the function of increasing the viscosity of the preparation and stabilizing the emulsion. As a result, they are especially useful as a base in a cosmetic emulsion.

Examples of higher fatty acids include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of fluorine-based oils include perfluoropolyether, perfluorodecalin, and perfluorooctane.

### (E) Alcohols

Copolymers of the present invention may be used after being dispersed or dissolved in an alcohol. Because copolymers of the present invention have excellent affinity with alcohols, which are commonly used as a component in cosmetics, alcohols can also be used in a cosmetic formulation. One or more polyhydric alcohols and/or lower monohydric alcohols can be used. Examples of lower alcohols include ethanol, isopropanol, n-propanol, t-butanol, and sec-butanol. Ethanol is preferred. Examples of polyhydric alcohols include dihydric alcohols such as 1,3-propanediol, 1,3-butylene glycol, 1,2-butylene glycol, propylene glycol, trimethylene glycol, tetramethylene glycol, 2,3-butylene glycol, pentamethylene glycol, 2-butene-1,4-diol, dibutylene glycol, pentyl glycol, hexylene glycol, and octylene glycol; trihydric alcohols such as glycerin, trimethylolpropane, and 1,2,6-hexanetriol; tetrahydric alcohols and higher such as pentaerythritol and xylitol; and sugar alcohols such as sorbitol, mannitol, maltitol, maltotriose, sucrose, erythritol, glucose, fructose, starch degradation products, maltose, xylitolose, and starch degraded sugar reduced alcohols. Examples other than these lower polyhydric alcohols include polyhydric alcohol polymers such as diethylene glycol, dipropylene glycol, triethylene glycol, polypropylene glycol, tetraethylene glycol, diglycerin, polyethylene glycol, triglycerin, tetraglycerin, and polyglycerin. Among these, ethanol, 1,3-propanediol, 1,3-butylene glycol, sorbitol, dipropylene glycol, glycerin, and polyethylene glycol are especially preferred.

The second aspect of the present invention relates to a cosmetic containing the film-forming agent for a cosmetic described above. A cosmetic of the present invention contains various ingredients for cosmetics in addition to the film-forming agent for a cosmetic described above. A film-forming agent containing components (D) and (E) can be blended into a cosmetic or the copolymer can be blended separately as the film-forming agent with other cosmetic ingredients. Components (D) and (E) explained above in reference to a film-forming agent of the present invention can then be used as additional ingredients for cosmetics. Other cosmetic ingredients include components (D) and (E) described above, (F) a surfactant, (G) a powder or colorant, (H) a thickener or gelling agent, (I) an organically modified clay mineral, (J) a silicone resin, (K) a silicone gum, (L) a silicone elastomer, (M) an organically modified silicone, (N) a UV protection component, (O) a water-soluble polymer, and water.

A cosmetic containing a film-forming agent of the present invention can include (F) a surfactant as an optional component. Depending on the intended use, the (F) surfactant can be one or more surfactants selected from a group consisting of (F1) a silicone-based surfactant, (F2) an anionic surfactant, (F3) a cationic surfactant, (F4) a nonionic surfactant, (F5) an amphoteric surfactant, and (F6) a semipolar surfactant.

Examples of (F1) silicone-based surfactants include polyglyceryl-modified silicones, diglyceryl-modified silicones, glyceryl-modified silicones, sugar-modified silicones, fluorinated polyether-modified silicones, polyether-modified silicones, carboxylic acid-modified silicones, linear silicone/polyether block copolymers (polysilicone-13, etc.), long-chain alkyl/polyether co-modified silicones, polyglyceryl-modified silicone elastomers, diglyceryl knitted elastomers, glyceryl-modified elastomers, and polyether modified elastomers. The silicones and elastomers described above provided with an alkyl branch, a linear silicone branch or a siloxane dendrimer branch at the same time as the hydrophilic group, if necessary, can also be used. Commercially available products include SH 3771 M, SH 3772 M, SH 3773 M, SH 3775 M, BY 22-008 M, BY 11-030, ES-5373 FORMULATION AID, ES-5612 FORMULATION AID, ES-5300 FORMULATION AID, and ES-5600 SILICONE GLYCEROL EMULSIFIER (all from Dow Corning Toray).

Examples of (F2) anionic surfactants include saturated or unsaturated fatty acid salts (such as sodium laurate, sodium stearate, sodium oleate, and sodium linolenate), alkyl sulfates, alkylbenzenesulfonic acids (such as hexylbenzenesulfonic acid, octylbenzenesulfonic acid, and dodecylbenzenesulfonic acid) and salts thereof, polyoxyalkylene alkyl ether sulfates, polyoxyalkylene alkenyl ether sulfates, polyoxyethylene alkyl sulfates, alkyl sulfosuccinates, polyoxyalkylene sulfosuccinate alkyl ester salts, polyoxyalkylene alkyl phenyl ether sulfates, alkane sulfonates, octyltrimethylammonium hydroxide, dodecyltrimethylammonium hydroxide, alkyl sulfonates, polyoxyethylene alkyl phenyl ether sulfates, polyoxyalkylene alkyl ether acetates, alkyl phosphates, polyoxyalkylene alkyl ether phosphate, acyl glutamate, α-acyl sulfonates, alkyl sulfonates, alkyl allyl sulfonates, α-olefin sulfonate, alkyl naphthalene sulfonates, alkane sulfonates, alkyl or alkenyl sulfates, alkyl amide sulfate, alkyl or alkenyl phosphates, alkylamidophosphates, alkyloylalkyl taurine salts, N-acyl amino acid salts, sulfosuccinate, alkyl ether carboxylate, amide ether carboxylate, α-sulfo fatty acid ester salt, alanine derivatives, glycine derivatives, and arginine derivatives. Salts include alkali metal salts such as sodium salts, alkaline earth metal salts such as magnesium salts, alkanolamine salts such as triethanolamine salts, and ammonium salts.

Examples of (F3) cationic surfactants include alkyl trimethyl ammonium chloride, stearyl trimethyl ammonium chloride, lauryl trimethyl ammonium chloride, cetyl trimethyl ammonium chloride, alkyl trimethyl ammonium chloride tallow, behenyl trimethyl ammonium chloride, stearyl trimethyl ammonium bromide, behenyl trimethyl ammonium bromide, distearyl dimethyl ammonium chloride, dicocoyl dimethyl ammonium chloride, dioctyl dimethyl ammonium chloride, di (POE) oleyl methyl ammonium chloride (2EO), benzalkonium chloride, alkyl benzalkonium chloride, alkyl dimethyl benzalkonium chloride, benzethonium chloride, stearyl dimethyl benzyl ammonium chloride, lanolin-derived quaternary ammonium salts, diethyl aminoethylamide stearate, dimethyl aminopropylamide stearate, amidopropyl dimethyl hydroxypropyl ammonium behenate chloride, stearoylcholaminoformyl methyl pyridinium chloride, cetylpyridinium chloride, tall oil alkylbenzylhydroxyethyl imidazolinium chloride, and benzyl ammonium salt.

Examples of (F4) nonionic surfactants include polyglyceryl diisostearate or diglyceryl polyhydroxystearate, isostearyl glyceryl ether, polyoxyalkylene ethers, polyoxyalkylene alkyl ethers, polyoxyalkylene fatty acid esters, polyoxyalkylene fatty acid diesters, polyoxyalkylene resin acid esters, polyoxyalkylene (hardened) castor oils, polyoxyalkylene alkylphenols, polyoxyalkylene alkyl phenyl ethers, polyoxyalkylene phenyl ethers, polyoxyalkylene alkyl esters, polyoxyalkylene alkyl esters, sorbitan fatty acid esters, polyoxyalkylene sorbitan alkyl esters, polyoxyalkylene sorbitan fatty acid esters, polyoxyalkylene sorbite fatty acid esters, polyoxyalkylene glycerin fatty acid esters, polyglycerin alkyl ethers, polyglycerin fatty acid esters, sucrose fatty acid esters, fatty acid alkanolamides, alkyl glucosides, polyoxyalkylene fatty acid bisphenyl ethers, polypropylene glycol, diethylene glycol, polyoxyethylene/polyoxypropylene block polymers, alkyl polyoxyethylene/polyoxypropylene block polymer ethers, polyoxyethylene/polyoxypropylene block polymers, alkyl polyoxyethylene/polyoxypropylene block polymer ethers, and fluorine-based surfactants.

Examples of (F5) amphoteric surfactants include imidazoline-type, amidobetaine-type, alkylbetaine-type, alkylamidobetaine-type, alkylsulfobetaine-type, amidesulfobetaine-type, hydroxysulfobetaine-type, carbobetaine-type, phosphobetaine-type, aminocarboxylic acid-type, and amidoamino acid-type amphoteric surfactants. Specific examples include imidazoline-type amphoteric surfactants such as 2-undecyl-N,N,N- (hydroxyethyl carboxymethyl)-2-imidazoline sodium and 2-cocoyl-2-imitazolinium hydroxide-1-carboxyethyloxy disodium salts; alkyl betaine-type amphoteric surfactants such as betaine lauryl dimethylaminoacetate and myristyl betaine; amidobetaine-type amphoteric surfactants such as coconut oil fatty acid amidopropyl dimethylaminoacetic acid betaine, palm kernel oil fatty acid amidopropyl dimethylaminoacetic acid betaine, beef tallow fatty acid amidopropyl dimethylaminoacetic acid betaine, hardened tallow fatty acid amidopropyl dimethylaminoacetic acid betaine, betaine laurate amidopropyl dimethylaminoacetate, betaine myristic acid amidopropyl dimethylaminoacetate, betaine amidopropyl dimethylaminoacetate palmitate, betaineamide dimethyl dimethylaminoacetate acetate, and amidopropyl oleate dimethylaminoacetic acid betaine; alkylsulfobetaine-type amphoteric surfactants such as coconut oil fatty acid dimethylsulfopropylbetaine; alkylhydroxysulfobetaine-type amphoteric surfactants such as lauryldimethyl aminohydroxysulfobetaine; and amidoamino acid-type amphoteric surfactants such as sodium N-lauroyl-N'-hydroxyethyl-N'-carboxymethylethylenediamine, sodium N-oleoyl-N'-hydroxyethyl-N'-carboxymethylethylenediamine, sodium N-cocoyl-N'-hydroxyethyl-N'-carboxymethylethylenediamine, potassium N-lauroyl-N'-hydroxyethyl-N'-carboxymethylethylenediamine, potassium N-oleoyl-N'-hydroxyethyl-N'-carboxymethylethylenediamine, sodium N-lauroyl-N-hydroxyethyl-N'-carboxymethylethylenediamine, sodium N-oleoyl-N-hydroxyethyl-N'-carboxymethylethylenediamine, sodium N-cocoyl-N-hydroxyethyl-N'-carboxymethylethylenediamine, monosodium N-lauroyl-N-hydroxyethyl-N ', N'-dicarboxymethylethylenediamine, monosodium N-oleoyl-N-hydroxyethyl-N ', N'-dicarboxymethylethylenediamine, monosodium N-cocoyl-N-hydroxyethyl-N ', N'-dicarboxymethylethylenediamine, disodium N-lauroyl-N-hydroxyethyl-N ', N'-dicarboxymethylethylenediamine, disodium N-oleoyl-N-hydroxyethyl-N ', N'-dicarboxymethylethylenediamine, and disodium N-cocoyl-N-hydroxyethyl-N ', N'-dicarboxymethylethylenediamine.

Examples of (F6) semipolar surfactants include alkylamine oxide-type surfactants, alkylamine oxides, alkylamidoamine oxides, and alkyl hydroxyamine oxides. Alkyl dimethylamine oxides having from 10 to 18 carbon atoms and alkoxyethyl dihydroxyethylamine oxides having from 8 to 18 carbon atoms are preferred. Specific examples include dodecyl dimethylamine oxide, dimethyloctylamine oxide, diethyldecylamine oxide, bis-(2-hydroxyethyl) dodecylamine oxide, dipropyltetradecylamine oxide, methylethylhexadecylamine oxide, dodecylamidopropyldimethylamine oxide, cetyl dimethylamine oxide, stearyl dimethylamine oxide, tallow dimethylamine oxide, dimethyl-2-hydroxyoctadecylamine oxide, lauryl dimethylamine oxide, myristyl dimethylamine oxide, stearyl dimethylamine oxide, isostearyl dimethylamine oxide, coconut fatty acid alkyldimethylamine oxide, amidopropyl dimethylamine oxide caprylate, amidopropyl dimethylamine oxide caprate, lauric acid amidopropyl dimethylamine oxide, myristate amidopropyldimethylamine oxide, amidopropyldimethylamine palmitate, amidopropyldimethylamine oxide stearate, amidopropyldimethylamine oxide isostearate, oleic acid amidopropyldimethylamine oxide, ricinoleic acid amidopropyl dimethylamine oxide, 12-hydroxystearic acid amidopropyldimethylamine oxide, coconut fatty acid amidopropyl dimethylamine oxide, palm kernel oil fatty acid amidopropyl dimethylamine oxide, castor oil fatty acid amidopropyl dimethylamine oxide, lauric acid amidoethyl dimethylamine oxide, myristate amidoethyl dimethylamine oxide, coconut fatty acid amidoethyl dimethylamine oxide, lauric acid amidoethyl diethylamine oxide, myristate amidoethyl diethylamine oxide, coconut fatty acid amidoethyl diethylamine oxide, lauric acid amide ethyl dihydroxyethylamine oxide, myristic acid amidoethyl dihydroxyethylamine oxide, and coconut fatty acid amidoethyl dihydroxyethylamine oxide.

There are no particular restrictions on the amount of the surfactant (F) in a film-forming agent or cosmetic of the present invention. However, in order to stabilize the film-forming agent or cosmetic, it can be blended into the film-forming agent or cosmetic in a range from 0.05 to 90% by weight, preferably 0.1 to 50% by weight, and more preferably 0.5 to 25% by weight.

### (G) Powders or Colorants

A film-forming agent or cosmetic of the present invention can be blended with a powder or colorant, especially a powder commonly used in cosmetic products (including powders and pigments used as colorants). A powder or colorant commonly used in cosmetics can be used without regard to shape (spherical, rod-like, acicular, tabular, sheet-like, irregular, spindle-shaped, bowl-shaped, raspberry-shaped, etc.), particle size (mist, fine particles, pigment grade, etc.), or particle structure (porous, non-porous, secondary aggregate, etc.). When these powders and/or colorants are used as a pigment, one or more selected from inorganic pigment powders, organic pigment powders, and resin powders with an average particle diameter in a range from 1 nm to 20 µm is preferred.

Examples of powders and pigments include inorganic powders, organic powders, surfactant metal salt powders (metal soaps), colored pigments, pearly pigments, metal powder pigments, and silicone elastomer powders. Compounds of these can also be used. These powders and colorants can also function as a UV protection component.

Specific examples include inorganic powders such as titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, red mica, biotite, lithium mica, silicic acid, silicic anhydride, aluminum silicate, sodium silicate, sodium magnesium silicate, magnesium silicate, aluminum magnesium silicate, calcium silicate, barium silicate, strontium silicate, metal tungstate, hydroxyapatite, vermiculite, higilite, bentonite, montmorillonite, hectorite, zeolite, ceramic powder, dibasic calcium phosphate, alumina, aluminum hydroxide, and boron nitride; organic powders such as polyamide powder, polyester powder, polyethylene powder, polypropylene powder, polystyrene powder, polyurethane powder, benzoguanamine powder, polymethylbenzoguanamine powder, polytetrafluoroethylene powder, polymethyl methacrylate powder, cellulose, silk powder, nylon powder, 12 nylon, 6 nylon, silicone powder, silicone rubber powder, silicone elastomer spherical powder coated with polymethylsilsesquioxane, polymethylsilsesquioxane spherical powder, styrene/acrylic acid copolymers, divinylbenzene/styrene copolymers, vinyl resins, urea resins, phenolic resins, fluororesins, silicon resins, acrylic resins, melamine resins, epoxy resins, polycarbonate resins, microcrystalline fiber powders, starch powder, and lauroyl lysine; surfactant metal salt powders such as zinc stearate, aluminum stearate, calcium stearate, magnesium stearate, zinc myristate, magnesium myristate, zinc palmitate, zinc laurate, zinc cetyl phosphate, calcium cetyl phosphate, and sodium zinc cetyl phosphate; colored pigments including inorganic red pigments such as red iron oxide, iron oxide, iron hydroxide and iron titanate, inorganic brown pigments such as γ-iron oxide, inorganic yellow pigments such as yellow iron oxide and loess, inorganic black pigments such as black iron oxide and carbon black, inorganic violet pigments such as manganese violet and cobalt violet, inorganic green pigments such as chromium hydroxide, chromium oxide, cobalt oxide and cobalt titanate, inorganic blue pigments such as navy blue and ultramarine blue, lake tar-based pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206 and Orange No. 207, and lake natural pigments such as carminic acid, raccaic acid, cartamine, bradylin and crocin; pearly pigments such as titanium oxide coated mica, titanium mica, iron oxide treated titanium mica, titanium oxide coated mica, bismuth oxychloride, bismuth oxychloride coated with titanium oxide, talc coated with titanium oxide, fish scale guanine, and titanium oxide-coated colored mica; and metal powder pigments such as metal powders of aluminum, gold, silver, copper, platinum, and stainless steel.

Silicone elastomer powders are the powdery component of the (L) silicone elastomers described below. These are crosslinked products of linear diorganopolysiloxanes consisting primarily of diorganosiloxy units (D units). These can be obtained by conducting a crosslinking reaction on an organohydrogenpolysiloxane having a silicon-bonded hydrogen atom in a side chain or at the terminal end and a diorganopolysiloxane having an unsaturated hydrocarbon group such as an alkenyl group in a side chain or at the terminal end in the presence of a hydrosilylation reaction catalyst. Silicone elastomer powders are softer and more elastic than silicone resin powders consisting of T units and Q units. Because they have excellent oil absorptivity, they can absorb oil on the skin and prevent makeup disintegration.

The silicone elastomer powder can take various shapes such as a spherical shape, a flat shape, or an irregular shape. The silicone elastomer powder may be in the form of an oil dispersion. The cosmetic of the present invention can use a silicone elastomer powder in the form of particles in which the primary particle diameter and/or average primary particle diameter measured using the laser diffraction/scattering method under observation using an electron microscope is within the range of 0.1 to 50 µm. A silicone elastomer powder having a primary particle with a spherical shape can be effectively blended. The silicone elastomer constituting the silicone elastomer powder preferably has a hardness of 80 or less, and more preferably 65 or less, as measured using a type-A durometer in accordance with JIS K6253, "Testing methods for the hardness of vulcanized rubbers and thermoplastic rubbers".

The silicone elastomer powder can be used in a cosmetic of the present invention in a form of an aqueous dispersion. Commercially available products of these aqueous dispersions include BY29-129 and PF-2001 PIF Emulsion from Dow Corning Toray.

The silicone elastomer powder can be surface treated with a silicone resin or silica. Examples of surface treatments are described in JP H02-243612 A, JP H08-12545 A, JP H08-12546 A, JP H08-12524 A, JP H09-241511 A, JP H010-36219 A, JP H011-193331 A, and JP 2000-281523 A. Another example of silicone elastomer powders are the cross-linked silicone powders listed in the "Cosmetic Classifications and Compounding Component Standards". Commercially available silicone elastomer powders include, for example, Tolefill E-506S, Tolefill E-508, 9701 Cosmetic Powder, and 9702 Powder from Dow Corning Toray.

Some or all of the powder or the colorant is preferably subjected to a water-repellency treatment. This enables it to be compounded stably in the oil phase. The powders or colorants may be compounded, and can be subjected to surface treatment with an all-purpose oil, a silicone compound other than an organopolysiloxane copolymer of the present invention, a fluorine compound, or a surfactant.

Examples of other water-repellent treatments include treating the powder or colorant with various water-repellent surface treatment agents. Examples include organosiloxane treatments such as methyl hydrogen polysiloxane treatment, silicone resin treatment, silicone gum treatment, acrylic silicone treatment and fluorinated silicone treatment, metal soap treatments such as zinc stearate treatment, silane treatments such as silane coupling agent treatment and alkyl silane treatment, fluorine compound treatments such as perfluoroalkylsilane, perfluoroalkylphosphate ester salt or perfluoropolyether treatment, amino acid treatments such as N-lauroyl-L-lysine treatment, oil treatments such as squalane treatment, and acrylic treatments such as alkyl acrylate treatment. These treatments can be used alone or in combination.

The powder or colorant is preferably treated with another powder dispersant or surface treatment agent. The dispersion or surface treatment can be performed using a novel powder treatment agent or treatment method proposed by the present inventors in WO 2009/022621 A, JP 2011-148784 A, JP 2011-149017 A, JP 2011-246704 A, JP 2011-246705 A, JP 2011-246706 A, WO 2009/022621 A, WO 2011/049246 A, WO 2011/049248 A, and Japanese Patent Application No. 2011-286973. The powder or colorant may also be slurried using one of these novel powder treatment agents or treatment methods. Because these novel treatment agents improve performance, such as improving the unique feel and dispersion stability, combined use with the novel cosmetic materials of the present invention is expected to further improve the functionality, feel, and storage stability of cosmetics.

In addition, some or all of the powder or colorant may be subjected to hydrophilic treatment. This enables the powder or colorant to be blended in relation to the aqueous phase.

In addition, some or all of the powder or colorant may be subjected to hydrophobizing and hydrophilizing treatment. This can impart emulsifying properties to the powder itself. An example of a commercially available product is MZY-500SHE from Teica.

If necessary, one or more (G) powder or colorant can be used in a film-forming agent or cosmetic of the present invention. There are no particular restrictions on the amount, but they can be blended in a range from 0.1 to 99.5% by mass, and preferably from 1 to 99% by mass, relative to the entire film-forming agent or cosmetic. The blending amount in the case of a solid powder cosmetic is preferably in a range from 80 to 99% by mass relative to the entire cosmetic.

### (H) Gelling or Thickening Agents

The gelling agent is preferably oil soluble. Specific examples include metal soaps such as aluminum stearate, magnesium stearate and zinc myristate, amino acid derivatives such as N-lauroyl-L-glutamic acid and α, γ-di-n-butylamine, dextrin fatty acid esters such as dextrin palmitate, dextrin stearate and dextrin 2-ethylhexanoate palmitate, Sucrose fatty acid esters such as sucrose palmitate and sucrose stearate, and benzylidene derivatives of sorbitol such as monobenzylidene sorbitol and dibenzylidene sorbitol. These can be used alone or in combinations of two or more as needed.

### (I) Organic Modified Clay Minerals

Examples of organic modified clay minerals include dimethylbenzyl dodecylammonium montmorillonite clay, dimethyl dioctadecyl ammonium montmorillonite clay, dimethyl alkyl ammonium hectorite, benzyl dimethyl stearyl ammonium hectorite, and aluminum magnesium silicate treated with distearyl dimethyl ammonium chloride. Commercially available products include Benton 27 (benzyldimethyl stearyl ammonium chloride treated hectorite from National Red) and Benton 38 (distearyl dimethyl ammonium chloride treated hectorite from National Red).

### (J) Silicone Resins

Silicone resins are organopolysiloxanes with a highly branched, reticular or cage structure, and are liquid or solid at room temperature. Any silicone resin commonly used in cosmetics can be used as long as it does not impair the object of the present invention. Solid silicone resins include MQ resins, MDQ resins, MTQ resins, MDTQ resins, TD resins, TQ resins and TDQ combining monoorganosiloxy units (M units) (where the organo groups are only methyl groups or methyl groups and vinyl groups or phenyl groups), diorganosiloxy units (D units) (where the organo groups are only methyl groups or methyl groups and vinyl groups or phenyl groups), triorganosiloxy units (T units) (where the organo groups are methyl groups, vinyl groups or phenyl groups), and siloxy units (Q units). Examples include trimethylsiloxysilicic acid, polyalkylsiloxysilicic acid, dimethylsiloxy unit-containing trimethylsiloxysilicic acid, and alkyl (perfluoroalkyl) siloxysilicic acid. These silicone resins are oil-soluble, and those that can be dissolved in D4 and D5 are preferred. Silicone resins form a uniform film when applied to skin and hair to provide protection against drying out and against low temperatures. Silicone resins with these branch units adhere firmly to skin and hair, and impart gloss and translucence to the skin and hair.

### (K) Silicone Gums

In the present invention, an organopolysiloxane with an ultrahigh viscosity of 1,000,000 mm²/s or more is referred to as a silicone gum, but can be used as a silicone oil. Silicone gums are linear diorganopolysiloxane with a very high degree of polymerization, and are also known as raw silicone gums or organopolysiloxane gums. Silicone gums are distinguished from oily silicones in that they have a measurable degree of plasticity due to their high degree of polymerization. Examples of raw silicone gums include substituted or unsubstituted organopolysiloxanes with dialkylsiloxy units (D units) dimethylpolysiloxane, methylphenylpolysiloxane, aminopolysiloxane, and methylfluoroalkyl polysiloxane, or those with a finely crosslinked structure of these. A typical example is a compound represented by the general formula: R¹⁰(CH₃)₂SiO{(CH₃)₂SiO}ₛ{(CH₃)R¹¹SiO}ₜSi(CH₃)₂R¹⁰. (In this formula, R¹¹ is a group selected from among a vinyl group, a phenyl group, an alkyl group having from 6 to 20 carbon atoms, an aminoalkyl group having from 3 to 15 carbon atoms, a perfluoroalkyl group having from 3 to 15 carbon atoms, and quaternary ammonium base-containing alkyl group having from 3 to 15 carbon atoms, a terminal R¹⁰ is a group selected from among an alkyl group having from 1 to 8 carbon atoms, a phenyl group, a vinyl group, an aminoalkyl group having from 3 to 15 carbon atoms, a hydroxyl group and alkoxy group having from 1 to 8 carbon atoms, s = 2,000 to 6,000, t = 0 to 1,000, and s + t = 2,000 to 6,000.) Among these, a dimethylpolysiloxane raw gum with a degree of polymerization from 3,000 to 20,000 is preferred. These silicone gums can be added to a film-forming agent or cosmetic of the present invention directly or in the form of a liquid gum dispersion dispersed in oily silicone (oil dispersion of silicone gum).

Examples of raw silicone gums include substituted or unsubstituted organopolysiloxanes with dialkylsiloxy units (D units) dimethylpolysiloxane, methylphenylpolysiloxane, aminopolysiloxane, and methylfluoroalkyl polysiloxane, or those with a finely crosslinked structure of these. A typical example is a compound represented by the general formula: R¹⁰(CH₃)₂SiO{(CH₃)₂SiO}ₛ{(CH₃)R¹²SiO}ₜSi(CH₃)₂R¹⁰. (In this formula, R¹² is a group selected from among a vinyl group, a phenyl group, an alkyl group having from 6 to 20 carbon atoms, an aminoalkyl group having from 3 to 15 carbon atoms, a perfluoroalkyl group having from 3 to 15 carbon atoms, and quaternary ammonium base-containing alkyl group having from 3 to 15 carbon atoms, a terminal R¹⁰ is a group selected from among an alkyl group having from 1 to 8 carbon atoms, a phenyl group, a vinyl group, an aminoalkyl group having from 3 to 15 carbon atoms, a hydroxyl group and alkoxy group having from 1 to 8 carbon atoms, s = 2,000 to 6,000, t = 0 to 1,000, and s + t = 2,000 to 6,000.) An amino-modified methylpolysiloxane raw gum having, for example, a 3-aminopropyl group or N-(2-aminoethyl) 3-aminopropyl group in a side chain or on a terminal end of the molecule is preferred. In the present invention, these silicone gums can be used alone or in combinations of two or more as needed.

Because silicone gums have a very high degree of polymerization, they form a long-lasting protective film on skin and hair with excellent breathability. This enables gloss and luster to be imparted to skin and hair, and imparts texture and firmness to skin and hair during and after use.

The amount of silicone gum added can be in a range from 0.05 to 30% by weight (mass), preferably in a range from 1 to 15% by weight (mass)% relative to the entire film-forming agent or cosmetic. If a silicone gum is used in the form of an emulsified composition prepared beforehand using an emulsifying step (such as emulsion polymerization), it can be more easily and stably blended into a film-forming agent or cosmetic of the present invention. If the amount of silicone gum is below the lower limit, gloss may be insufficiently imparted to skin and hair.

### (L) Silicone Elastomers

Silicone elastomers can be blended with a film-forming agent or cosmetic in any form depending on the intended purpose. In addition to the silicone elastomer powders described in (G) Powders or Colorants section above, mixing a silicone elastomer in the form of a crosslinkable organopolysiloxane is preferred. A silicone elastomer powder can also be used in a cosmetic of the present invention in the form of an aqueous dispersion. Commercially available aqueous dispersions include, for example, BY 29-129 and PF-2001 PIF Emulsion from Dow Corning Toray. Blending in these silicone elastomer powders in the form of an aqueous dispersion (that is, a suspension) is extremely useful from the standpoint of further improving the feel of a cosmetic of the present invention when used.

Non-emulsifiable crosslinkable organopolysiloxanes having a structure in which an organopolysiloxane chain is three-dimensionally crosslinked in a reaction with a crosslinkable component and not having hydrophilic components such as polyoxyalkylene units are preferred. These crosslinkable organopolysiloxanes can be used without restriction, regardless of the manufacturing method such as dilution and physical form such as its properties. Especially preferred examples include the α, ω-diene crosslinked silicone elastomers described in US Pat. No. 5,654,362 (available commercially from Dow Corning of the US as DC 9040 Silicone Elastomer Blend, DC 9041 Silicone Elastomer Blend, DC 9045 Silicone Elastomer Blend, and DC 9046 Silicone Elastomer Blend. Crosslinkable organopolysiloxanes that have fluidity at room temperature can also be used. An example is 3901 LIQUID SATIN BLEND (Dow Corning of the US).

### (M) Organically Modified Silicones

These organically modified silicones are preferably lipophilic. Specific examples include amino-modified silicone, amino polyether-modified silicone, epoxy-modified silicone, carboxyl-modified silicone, amino acid-modified silicone, carbinol-modified silicone, acrylic-modified silicone, phenol-modified silicone, amidoalkyl-modified silicone, aminoglycol-modified silicone, and alkoxy-modified silicone. In addition to a polysiloxane bond as a main chain, these organically modified silicones may have an alkylene chain, an aminoalkylene chain or a polyether chain to the extent that the compound does not have hydrophilicity. The organic modifying group may be in a side chain and/or on a terminal end of the polysiloxane chain. When a cosmetic of the present invention is a hair care product, an amino-modified silicone, carbinol-modified silicone, amino polyether-modified silicone or amino glycol-modified silicone can be used. An example is an amino-modified silicone having a 3-aminopropyl group or an N-(2-aminoethyl) 3-aminopropyl group.

The following is a description of higher alkyl-modified silicones, alkyl-modified silicone resins, and polyamide-modified silicone resins which are preferred examples of organically modified silicones. Higher alkyl-modified silicones are waxy at room temperature and are useful components as raw materials in cosmetics. As a result, they can be used advantageously in cosmetics of the present invention. Examples of higher alkyl modified silicone waxes include methyl long-chain alkylpolysiloxane capped at both ends of the molecular chain with a trimethylsiloxy group, dimethylpolysiloxane/methyl long-chain alkylsiloxane copolymers capped at both ends of the molecular chain with a trimethylsiloxy group, and long-chain alkyl-modified dimethylpolysiloxane capped at both ends of the molecular chain. Commercially available products include AMS-C30 Cosmetic Wax and 2503 Cosmetic Wax (from Dow Corning of the US).

In a cosmetic of the present invention, the higher alkyl-modified silicone wax preferably has a melting point of 60°C or more from the standpoint of longer lasting makeup and higher temperature stability.

Alkyl-modified silicone resins impart sebum resistance, moisturizing properties, and a fine texture to cosmetics. Those that are waxy at room temperature are preferred. A preferred example is the silsesquioxane resin wax described in JP 2007-532754 A. A commercially available product is SW-8005 C30 RESIN WAX (from Dow Corning of the US).

Examples of polyamide-modified silicones include, for example, the siloxane-based polyamide compounds described in U.S. Pat. 5981680 (JP 2000-038450 A) and JP 2001-512164 A. Commercially available products include 2-8178 Gellant and 2-8179 Gellant (from Dow Corning of the US). These polyamide-modified silicones also function as a thickening/gelling agent for oily raw materials, especially silicone oils.

### (N) UV Protection Components

UV protection components include organic UV protection components and inorganic UV protection components. When a cosmetic of the present invention is a sunscreen cosmetic, use of at least one type of organic or inorganic UV protection component is preferred, and use of an organic UV protection component is especially preferred. A film-forming agent of the present invention is usually compatible with poorly soluble organic ultraviolet protective components such as hexyl diethylaminohydroxybenzoyl benzoate (Ubinal A), bisethylhexyloxyphenol methoxyphenyl triazine (Tinosorb S), 2-cyano-3,3-diphenylpropa-2-enoic acid 2-ethylhexyl ester (Octocrylene), and cinnamic acid-based UV absorbers, and these can improve the blend stability of a film-forming agent of the present invention.

Inorganic ultraviolet protection components include inorganic pigment powders and metal powder pigments blended in the form of ultraviolet dispersants. Examples include metal oxides such as titanium oxide, zinc oxide, cerium oxide, lower titanium oxide and iron-doped titanium oxide, metal hydroxides such as iron hydroxide, metal flakes such as tabular iron oxide and aluminum flakes, and ceramics such as silicon carbide. Among these, at least one selected from among granular, tabular, acicular, or fibrous metal oxides and metal hydroxide fine particles with an average particle size in a range from 1 to 100 nm is preferred. These powders may be subjected to one or more surface treatments common in the art. Examples include fluorine compound treatments (preferably perfluoroalkyl phosphate treatment, perfluoroalkylsilane treatment, perfluoropolyether treatment, fluorosilicone treatment, and fluorinated silicone resin treatment), silicone treatments (preferably methyl hydrogen polysiloxane treatment, dimethyl polysiloxane treatment, and vapor phase tetramethyl tetrahydrogen cyclotetrasiloxane treatment), silicone resin treatment (preferably trimethylsiloxysilicic acid treatment), pendant treatments (a method in which alkyl chains etc. are added after gas phase silicone treatment), silane coupling agent treatments, titanium coupling agent treatments, silane treatments (preferably alkylsilane or alkylsilazane treatment), oil treatments, N-acylated lysine treatments, polyacrylic acid treatments, metal soap treatments (preferably with stearic acid or myristate), acrylic resin treatments, and metal oxide treatments. In another example, the surface of fine titanium oxide particles is treated with an alkylsilane after the surface of the fine particles has been coated with a metal oxide such as silicon oxide or alumina. The amount of surface treatment is preferably in a range from 0.1 to 50% by mass relative to the total mass of the powder.

Organic UV protection components are lipophilic UV protection components. Examples include benzoic acid-based UV absorbers such as p-aminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and hexyl diethylaminohydroxybenzoyl benzoate; anthranilic acid-based UV absorbers such as homomenthyl-N-acetylanthranilate; salicylic acid-based UV absorbers such as amyl salicylate, menthyl salicylate, homomenthyl salicylate, octyl salicylate, phenyl salicylate, benzyl salicylate, and p-isopropanol phenyl salicylate; cinnamic acid-based UV absorbers such as octylcinnamate, ethyl-4-isopropyl cinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, glyceryl mono-2-ethylhexanoyl-diparamethoxycinnamate, and 3-methyl-4-[methylbis (trimethylsiloxy) silyl] butyl 3,4,5-trimethoxycinnamate; benzophenone-based UV absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4methoxybenzophenone-5-sulfonate, 4-phenylbenzophenone, 2-ethylhexyl-4'-phenylbenzophenone-2-carboxylate, hydroxy-4-n-octoxybenzophenone, and 4-hydroxy-3-carboxybenzophenone; and others such as 3-(4'-methylbenzylidene)-d,l-camphor, 3-benzylidene-d,l-camphor, urocanic acid, urocanic acid ethyl ester, 2-phenyl-5-methylbenzoxazole, 2,2'-hydroxy-5-methylphenylbenzotriazole, 2-(2'-hydroxy-5'-t-octylphenyl) benzotriazole, 2-(2'-hydroxy-5'-methylphenyl) benzotriazole, dibenzalazine, dianisoylmethane, 4-methoxy-4'-t-butyldibenzoylmethane, and 5-(3,3-dimethyl-2-norbornylidene)-3-pentan-2-one.

These organic ultraviolet protection components can also be incorporated into a hydrophobic polymer powder. This polymer powder may be hollow, have an average primary particle size in a range from 0.1 to 50 µm, and have a broad or narrow particle size distribution. The polymer used here can be an acrylic resin, methacrylic resin, styrene resin, polyurethane resin, polyethylene, polypropylene, polyethylene terephthalate, silicone resin, nylon, acrylamide resin, or silylated polypeptide resin. A polymer powder containing an organic UV protective component in a range from 0.1 to 30% by mass is preferred, and a polymer powder containing the UV-A absorber 4-tert-butyl-4'-methoxydibenzoylmethane is especially preferred.

These organic ultraviolet protective component can also be dispersed in water. An example of such a commercially available product is Tinosorb A2B (from BASF).

In a cosmetic of the present invention, at least one type of ultraviolet ray protection component selected from a group consisting of fine particle titanium oxide, fine particle zinc oxide, 2-ethylhexyl paramethoxycinnamate, 4-tert-butyl-4'-methoxydibenzoylmethane, hexyl diethylaminohydroxybenzoyl benzoate, bisethylhexyloxyphenol methoxyphenyl triazine, 2-cyano-3,3-diphenylprop-2-enoic acid 2-ethylhexyl ester, and benzophenone UV absorbers can be used. These UV protection components are widely used and readily available, and have a high UV protection effect. A combination of organic and inorganic ultraviolet protection components is preferred, and a combination of an ultraviolet protection component corresponding to UV-A and an ultraviolet protection component corresponding to UV-B is especially preferred.

### (O) Water-Soluble Polymers

A film-forming agent or cosmetic of the present invention may also be an aqueous emulsion containing a large amount of water-soluble components, and (O) the water-soluble polymer preferably blended in depends on the formulation. One or more water-soluble polymers can be used. Examples of water-soluble polymers include plant polymers such as gum arabic, tragacanth gum, galactan, guar gum, carob gum, karaya gum, carrageenan, pectin, agar, quince seed (quince), algae colloid (brown algae extract), starch (rice, corn, potato, wheat), and glycyrrhizic acid; microbial polymers such as xanthan gum, dextran, succinoglucan, and pullulan; and animal polymers such as collagens, caseins, albumin, and gelatins. Examples of semi-synthetic water-soluble polymers include starch-based polymers such as carboxymethyl starch and methylhydroxypropyl starch; cellulosic polymers such as methylcellulose, nitrocellulose, ethylcellulose, methylhydroxypropylcellulose, hydroxyethylcellulose, cellulose sodium sulfate, hydroxypropylcellulose, sodium carboxymethylcellulose (CMC), crystalline cellulose, and cellulose powders; and alginic acid-based polymers such as sodium alginate and propylene glycol alginate. Examples of synthetic water-soluble polymers include vinyl polymers such as polyvinyl alcohol, polyvinyl methyl ether polymer, polyvinyl pyrrolidone, and carboxyvinyl polymer (CARBOPOL 940 and 941 from BF Goodrich), polyoxyethylene polymers such as polyethylene glycol 20,000, polyethylene glycol 6,000, and polyethylene glycol 4,000; copolymers such as polyoxyethylene polyoxypropylene copolymers and PEG/PPG methyl ether; acrylic polymers such as sodium polyacrylate, polyethyl acrylate, and polyacrylamide; polyethylene imines; and cationic polymers. Other cationic water-soluble polymers that can be used especially in hair care products include quaternary nitrogen-modified polysaccharides (cation-modified cellulose, cation-modified hydroxyethyl cellulose, cation-modified guar gum, cation-modified locust bean gum, cation-modified starch, etc.), dimethyldiallylammonium chloride derivatives (such as dimethyldiallylammonium chloride/acrylamide copolymers, polydimethylmethylenepiperidinium chloride, etc.), and vinylpyrrolidone derivatives (such as vinylpyrrolidone/dimethylaminoethyl methacrylic acid copolymer salts, vinylpyrrolidone/methacrylamidopropyl trimethylammonium chloride copolymers, vinylpyrrolidone/methylvinylimidazolium chloride copolymers, etc.).

Other components commonly used in the art can be added to a cosmetic of the present invention in a range that does not impair the effects of the invention. Examples include organic resins, moisturizers, preservatives, antibacterial agents, fragrances, salts, antioxidants, pH adjusters, chelating agents, cooling agents, anti-inflammatory agents, skin-beautifying ingredients (whitening agents, cell activators, skin roughness improving agents, blood circulation promoters, skin astringents, antiseborrheic agents, etc.), vitamins, amino acids, nucleic acids, hormones, and inclusion compounds. Specific examples are listed in paragraphs 0100 to 0113 of JP 2011-149017 A, but the present invention is not limited to these examples.

A cosmetic of the present invention may be blended with natural plant extract components, seaweed extract components, and herbal components depending on the intended use. Two or more of these components may also be used. Specific examples are listed in paragraph 0115 of JP 2011-149017 A, but the present invention is not limited to these examples.

A cosmetic of the present invention may be blended with a solvent such as a light isoparaffin, ether, LPG, N-methylpyrrolidone, or next-generation freon in addition to purified water or mineral water depending on the intended use.

In addition to a copolymer of the present invention, a cosmetic of the present invention may also include at least one type selected from the group consisting of acrylic silicone dendrimer copolymers and alkyl-modified silicone resin waxes. These are film-forming components like a copolymer of the present invention but, unlike a copolymer of the present invention, they do not improve washability. Therefore, these should be used in a range that does not impair the technical effects of the present invention.

A preferred example of an acrylic silicone dendrimer copolymers is the vinyl polymer with a carbosiloxane dendrimer structure in the side chain described in Japanese Patent No. 4,009,382 (JP 2000-063225 A). Examples of commercially available products include FA 4001 CM Silicone Acrylate and FA 4002 ID Silicone Acrylate from Dow Corning Toray.

A preferred example of an alkyl-modified silicone resin wax is the silsesquioxane resin wax described in JP 2007-532754 A.

A cosmetic of the present invention can take the form of a liquid, emulsion, cream, solid, paste, gel, powder, multilamellar emulsion, mousse, or spray.

A film-forming agent of the present invention has water resistance and sebum resistance, yet is also capable of forming a film on skin or hair with excellent washability. As a result, cosmetics with such a functional film can be designed.

Specific cosmetic products of the present invention include cosmetics for skin such as skin cleansing products, skin care products, makeup products, antiperspirant products, and UV protection products; cosmetics for hair such as hair cleansing products, hair styling products, hair coloring products, hair restoration products, hair rinse products, hair conditioner products, and hair treatment products; bath products; hair growth agents; hair restorers; analgesics; fungicides; anti-inflammatory agents; fresheners; and anti-aging agents for skin. However, the present invention is not restricted to these examples.

These cosmetics for skin can be used on the entire body or on the scalp, face (including lips, eyebrows and cheeks), hands, or fingernails. Specific examples include skin cleaning products such as cleansing gels, cleansing creams, cleansing foams, facial cleansing creams, eye makeup removers, facial cleansing foams, liquid soaps (body soaps), hand soaps, gel soaps, shaving cream, nail polish removers, and anti-acne cosmetics; skin care products such as skin creams, scalp treatments, skin milks, milk lotions, milky lotions, facial packs, body powders, essence, shaving lotions, and massage oils; makeup products such as foundations, liquid foundations, oily foundations, makeup bases, white powders, face powders, blush, lip balms, lipsticks and rouges, lip glosses, eye creams, mascaras, eyebrow cosmetics, and eyelash cosmetics; antiperspirants such as deodorants; and UV protection products such as sunscreen agents and sunburn agents (suntan agents).

Specific examples of cosmetics for hair include hair cleaning products such as shampoos and rinse-in shampoos; hair styling products such as hair waxes, hair curling agents, setting agents, hair creams, hair sprays, and hair liquids; hair coloring products such as hair dyes, hair color sprays, hair color rinses, and hair color sticks; hair restoration products such as hair tonics, hair treatment essences, and hair packs; and hair rinses and conditioning products such as oil rinses, cream rinses, treatment rinses, hair conditioners, and hair treatments. Bath cosmetics include bubble bath liquids.

Because film-forming agents of the present invention have all-purpose film-forming properties, they can be used in non-cosmetic products as well such as in external preparations, paints, coating agents, defoamers, and deodorants. Film-forming agents of the present invention can form a functional film on substrates with applications other than those of cosmetics, and are useful in such applications.

### Examples

The following is a more detailed description of the present invention with reference to examples. The present invention, however, is not restricted to these examples.

### 1. Preparation of Copolymers and Liquid Compositions Thereof

### Example 1

Isopropyl alcohol (IPA) was added (115.0 g) to a 500 ml four-necked flask equipped with a stirrer, a thermometer, and a reflux tube. Bubbling was performed with nitrogen gas to sufficiently degas the alcohol, and the alcohol was then heated to 80°C. Using a dropping funnel, 4.0 g of acrylic acid (5% by weight), 26.4 g of methyl methacrylate (33% by weight), 9.6 g of n-butyl acrylate (12% by weight), 40.0 g of a carbosiloxane dendrimer monomer (50% by weight) represented by Formula (A-1), 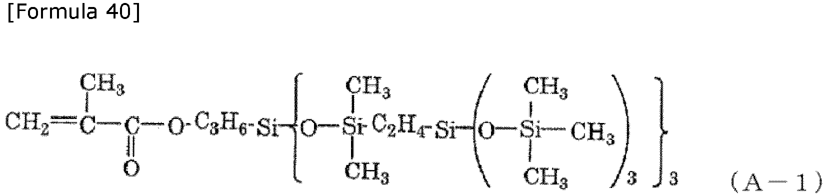 and 0.8 g of 2,2'-azobis-2-methylbutyronitrile (V-601 from Otsuka Chemical) were added and dissolved. Under a nitrogen atmosphere, a monomer mixture was added dropwise using the dropping funnel over the course of three hours while keeping the temperature at 80°C. After completion of the dropwise addition of the monomer, the mixture was heated and stirred under a nitrogen atmosphere for three hours. When the polymerization addition rate of the reaction product was analyzed after stirring using gas chromatography, the polymerization conversion rate was found to be 95%, and a vinyl polymer was obtained. GPC analysis revealed that the polymer had a number average molecular weight of 19,500. The isopropyl alcohol solution of the vinyl polymer was added to a rotary evaporator and stripped up to 10 mmHg at 160°C in order to obtain a solid. Caprylyl methicone (FZ-3196) was added to the resulting solid to obtain a cosmetic film-forming agent with a nonvolatile content of 39.4%.

### < Analysis >

Number average molecular weight Mn: This was analyzed using a standard polystyrene calibration curve with tetrahydrofuran serving as the elution solvent.

Viscosity measurement: The viscosity of the composition at 25°C was measured using a Viscomic EMD Type-E viscometer from Tokyo Keiki.

Nonvolatile component concentration: 1 g of sample was weighed on an aluminum dish with a diameter of 6 cm, and the concentration was determined using the remaining sample after heating at 150°C for one hour.

### < Evaluation >

Film hardness: 1 g of a sample was weighed on an aluminum dish with a diameter of 6 cm, and heated at 150°C for one hour to form a film. The aluminum dish was then bent to determine whether or not the film would crack.

Film stickiness: Film formed on an aluminum plate in the same manner described above was touched with a finger to determine whether or not the aluminum plate integrated with the film could be lifted.

Contact angle (water): After applying the IPA solution of the vinyl copolymer to a glass plate, the solvent was removed by drying at room temperature to obtain a coating film of the vinyl polymer. Next, a 5 µL drop of water was placed on the surface of the coating film, and the contact angle with the water was measured. The measurement device was a drop shape analysis system (KRUSS DSA10 Mk-2), and the average value of n ≤ 5 was used.

Contact angle (artificial sebum): After applying the IPA solution of the vinyl copolymer to a glass plate, the solvent was removed by drying at room temperature to obtain a coating film of the vinyl polymer. Next, a 5 µL drop of artificial sebum (triolein:oleic acid:squalane = 3:1:1) was placed on the surface of the coating film, and the contact angle with the artificial sebum was measured. The measurement device was a drop shape analysis system (KRUSS DSA10 Mk-2), and the average value of n ≤ 5 was used.

Washability Test 1: After adding 0.2 g of a 0.5 wt% toluene solution of Sudan Red III (CAS # 85-86-9) to 1 g of a FZ-3196 solution of the vinyl copolymer on a glass plate and mixing them together, the solvent was dried and removed for 1 hour at 80°C to obtain a colored vinyl polymer coating film. A 0.05 mol/L aqueous solution of potassium hydroxide was added to a beaker equipped with a magnetic stirrer, and the coating film was half immersed along with the glass plate. The solution was then stirred for three minutes. After three minutes, the glass plate was removed, washed with ion-exchanged water in a washing bottle, and air-dried. If the coating film immersed in the potassium hydroxide aqueous solution was removed by washing, the evaluation was NO. If the coating film remained and was not removed by washing, the evaluation was YES.

### Comparative Example 1

Commercially available solvent FA 4002 ID SILICONE ACRYLATE from Dow Corning (carboxysiloxane dendrimer with an acid value of 0 mgKOH/g and no acidic groups) was added to a rotary evaporator and stripped up to 10 mmHg at 160°C to obtain a solid. Caprylyl methicone (FZ-3196) was then added to the resulting solid to obtain a gel composition with a solid concentration of 42.5%.

### Comparative Example 2

As in Comparative Example 1, commercially available solvent FA 4004 ID SILICONE ACRYLATE from Dow Corning (carboxysiloxane dendrimer with an acid value of 0 mgKOH/g and no acidic groups) was added to a rotary evaporator and stripped up to 10 mmHg at 160°C to obtain a solid. Caprylyl methicone was then added to the resulting solid to obtain a liquid composition with a solid concentration of 40.5%.

### Examples 2-18, Comparative Examples 3-6

These were prepared in the same manner as Example 1 except that the monomer raw materials and weight percentages were changed as shown in Tables 1 to 6 below. The abbreviations used in the tables are as follows. In Example 18, the evaluation was performed using an equivalent amount of isopropyl alcohol (IPA) instead of caprylyl methicone.
Component (B):
   AA: Acrylic acid
   MAA: Methacrylic acid
Component (C):
   MMA: Methyl methacrylate
   n-Bu-A: n-Butyl acrylate
   2EHMA: 2-Ethylhexyl methacrylate
   ISA: Isostearyl acrylate
   SA: Stearyl acrylate
   SMA: Stearyl methacrylate
   BMA: Benzyl methacrylate

### Component (A):

### A-1:

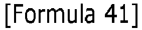

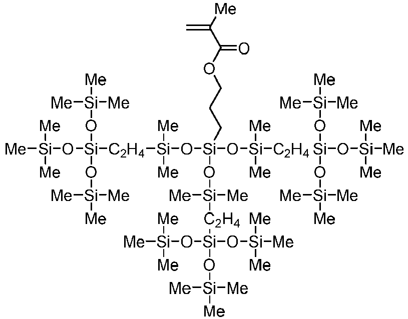

### A-2:

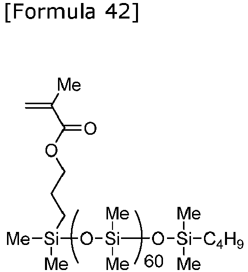

### A-3:

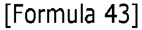

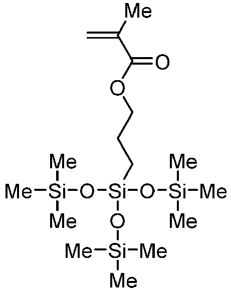

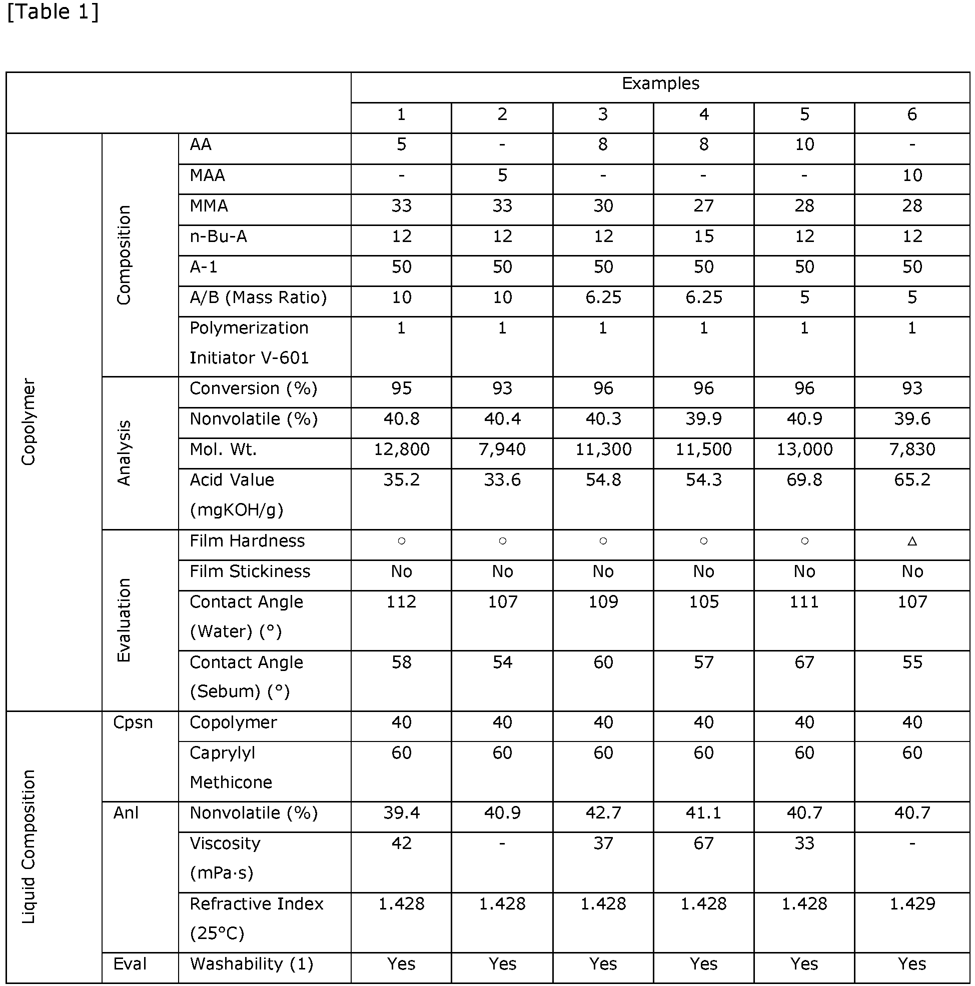

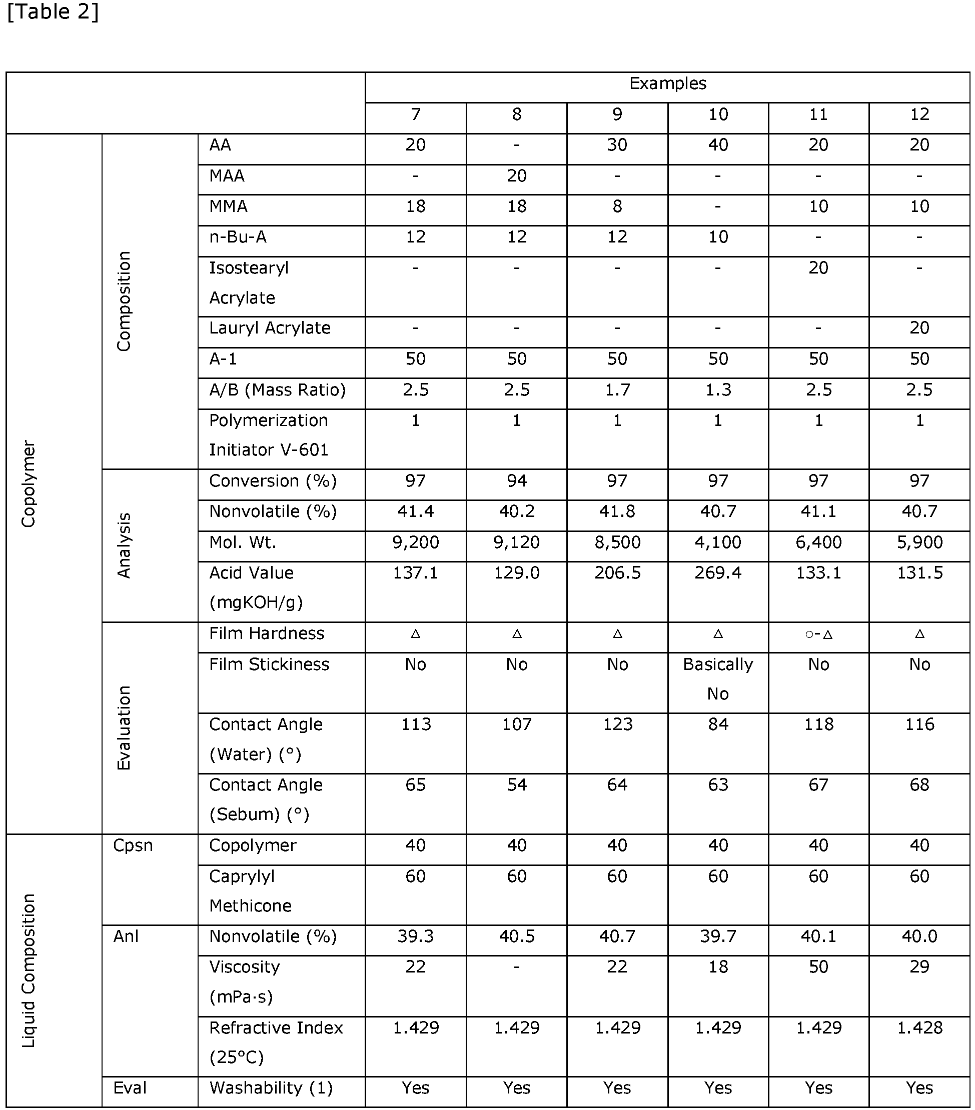

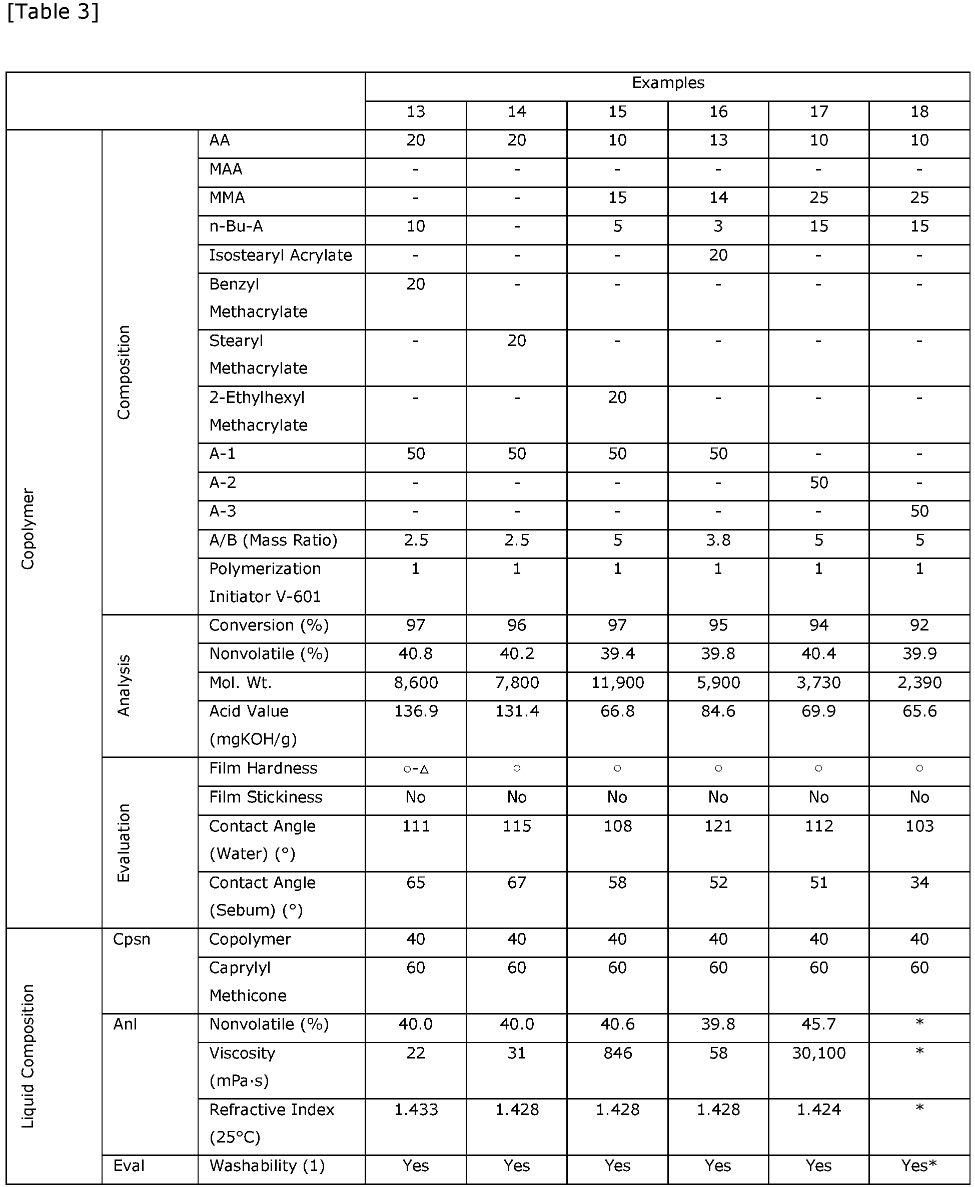

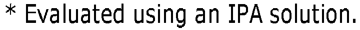

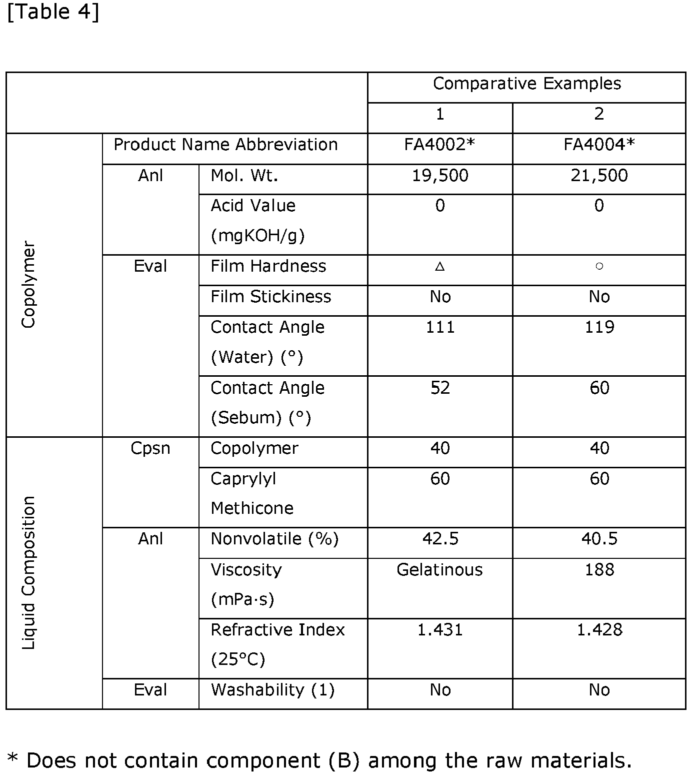

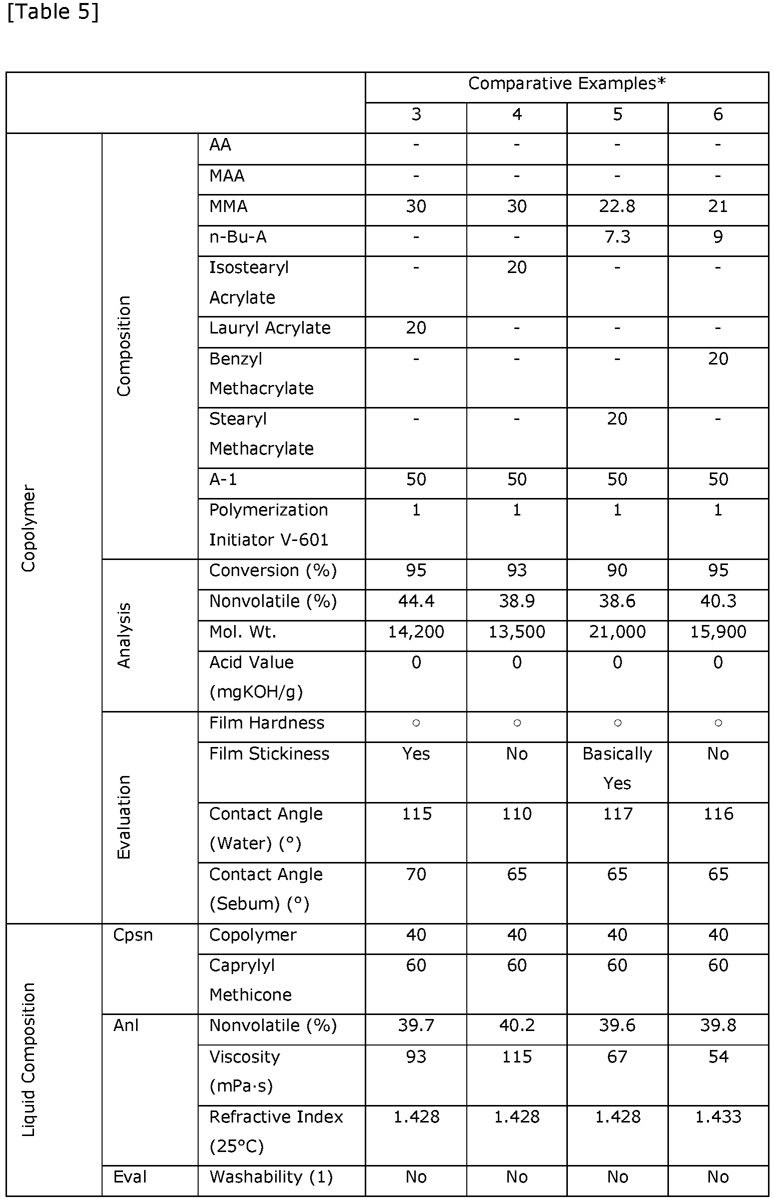

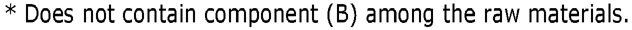

In addition to the results described above, all of the films exhibited the slippery feeling and smoothness unique to silicone. The copolymers of the present invention had sufficient film hardness, durability, and conformability to contours. They also were not sticky, had good water resistance and sebum resistance, and exhibited washability using ionic aqueous solutions.

### Washability Test 2

In order to confirm suitability for all-purpose use, the same test was performed on the composition in Example 4 except that a commercially available detergent, either "Additive-Free Foam" body soap from Miyoshi Soap or "Dove Beauty Moisture Cream" facial cleanser foam from Uni-Lever, was used instead of the 0.05 mol/L potassium hydroxide aqueous solution used in Washability Test 1. The immersed portion was washed as shown in FIG. 1. "Additive-Free Foam" body soap was used on the left and "Dove Beauty Moisture Cream" facial cleaner foam was used on the right in the photograph. Therefore, it is clear that the copolymer composition of the present invention is washable not only using a simple ionic aqueous solution but also using commercially available all-purpose cleansers.

### 2. Preparation of Powder Compositions

### Examples 19-26, Comparative Examples 7-11

After mixing together 70.8 parts by weight titanium oxide (Si-TiO2-CR50 from Miyoshi Kasei), 14.2 parts by weight red iron oxide (SA-Rouge Shichiho from Miyoshi Kasei), 5.0 parts by weight silicone surfactant (ES-5600 Silicone Glycerol Emulsifier from Dow Corning), 10.0 parts by weight FZ-3196 solution prepared in Examples 1-11 and Comparative Examples 1-5, and 7.0 parts by weight FZ-3196, a paste-like pigment composition was obtained using a three-roll mill (EXAKTM-50I).

### [Average Particle Size]

After the powder composition was diluted by a factor of 500 using D5 (SH245), the average particle size was measured with the method of cumulants using ELSZ-2000ZS (Otsuka Electronics).

### Washability Test 3

After applying the powder composition to a glass plate, the powder composition was dried at 50°C for 2 hours to obtain a powder coating film. A single drop of Biore u Foaming Hand Soap (Kao Corporation) was applied to the surface of the powder coating film, and then gently rubbed in by rotating a finger 10 times. A JK Wiper (Nippon Paper Crecia) was pressed against the applied soap, and checked to determine whether powder had adhered to the JK Wiper.

### Water Resistance

After applying the powder composition to a glass plate, the powder composition was dried at 50°C for 2 hours to obtain a powder coating film. A single drop ion-exchange water was applied to the surface of the powder coating film, and then gently rubbed in by rotating a finger 10 times. A JK Wiper was pressed against the applied soap, and checked to determine whether powder had adhered to the JK Wiper.

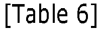

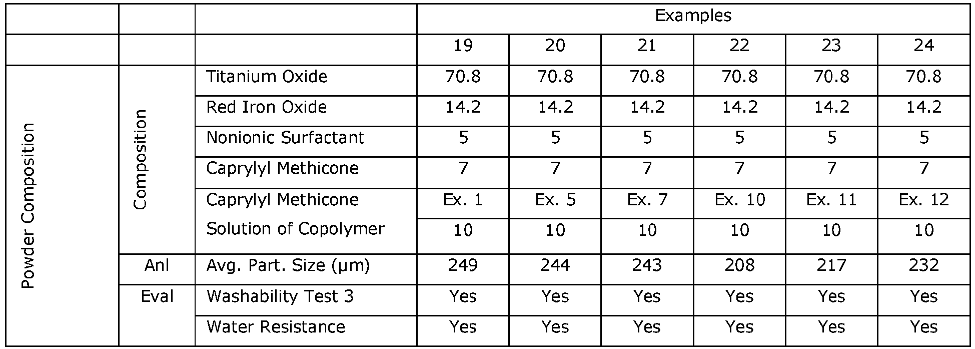

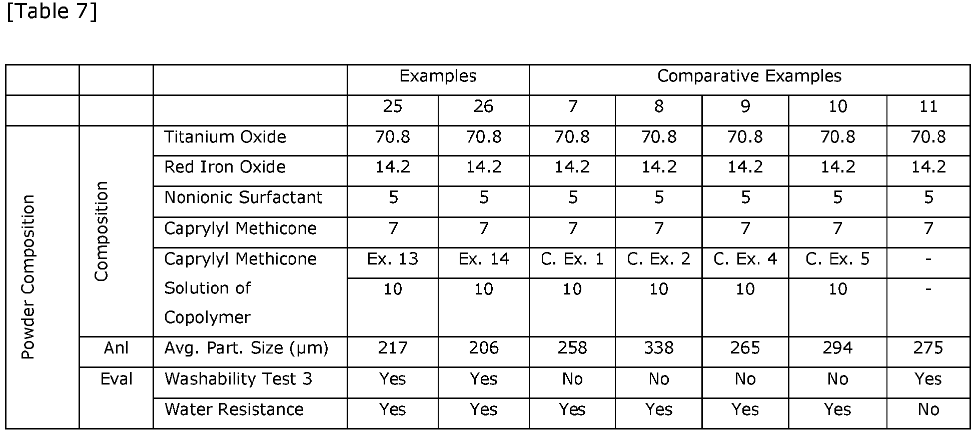

As seen above, a simple film of these examples had good water resistance and washability, and a film containing pigments used in cosmetics also had good water resistance and washability. In addition, the average particle size was smaller than that of film-forming agents without carboxylic acid, and dispersibility was excellent.

### Examples 27-29

### [Emulsification Evaluation]

The water-in-oil emulsion compositions shown in tables were prepared using the copolymer compositions obtained in Examples 2, 3, 9 and 10, and it was confirmed that emulsion compositions were obtained. The viscosity and results of observations under a microscope are also shown in the tables. In the tables, the parts are parts by weight (mass). The water-in-oil emulsion compositions shown in Table 10 were prepared using the copolymer compositions obtained in Examples 3, 8 and 16, and it was confirmed that emulsion compositions were obtained. The viscosity and results of observations under a microscope are also shown in Table 10. In the table, the parts are parts by weight (mass).

### [Preparation Method for Water-in-Oil Emulsion Compositions]

1. An oil (SH 200 2cst from Dow Corning) and a silicone-based surfactant (ES-5300 Formulation Aid from Dow Corning) were added to a 200-ml container.
2. Stirring was performed to uniformly disperse or dissolve the surfactant in the oil (Oil Phase A).
3. Table salt and ion-exchange water were added to a separate container and mixed with a spatula to dissolve the salt. Then, 1,3-butylene glycol was mixed in and dissolved (Aqueous Phase B).
4. The serrated blades of a Homo Disper were immersed in Oil Phase A, and the Aqueous Phase B was poured into the Oil Phase A at a constant rate over 45 seconds while stirring at 1000 rpm.
5. The speed of the Homo Disper was increased to 3,500 rpm and stirring was performed for two minutes to uniformly emulsify the contents.
6. Once stopped, the oil adhering to the inner wall of the container was scraped off and mixed into the resulting emulsion using a spatula.
7. The speed of the Homo Disper was increased to 3,500 rpm and stirring was performed for three minutes to uniformly emulsify the contents.

### [Viscosity Measurement]

The viscosity of the emulsion compositions was measured at 25°C using Viscomic EMD, an E-type viscometer from Tokyo Keiki Co., Ltd.

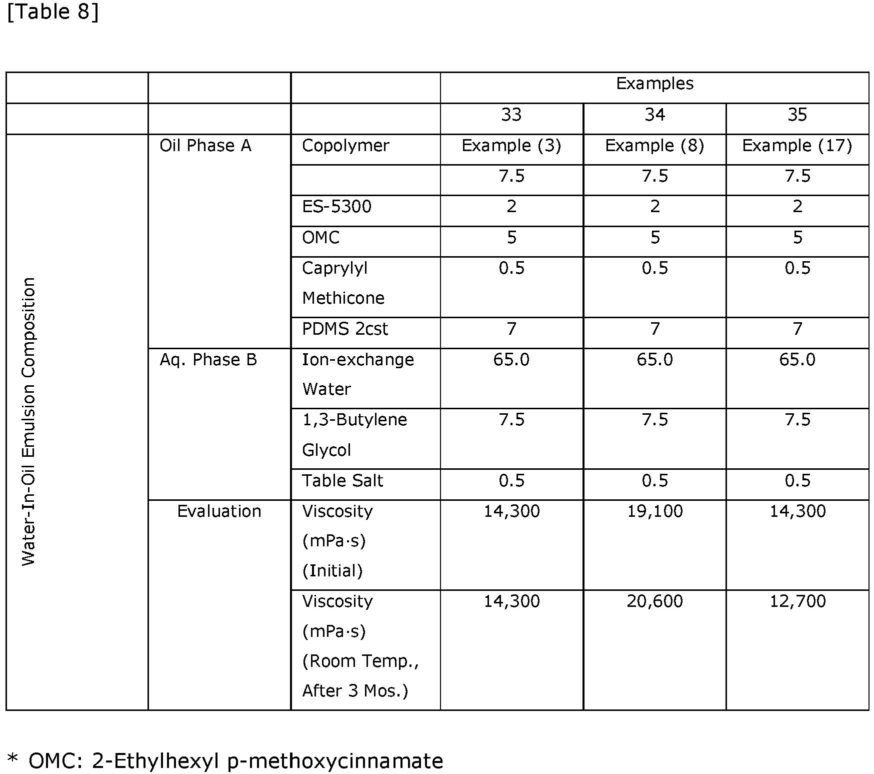

Water-in-oil emulsions containing a copolymer of the present invention were prepared in this manner and changes in the emulsions were observed over three months at room temperature. Because the viscosity hardly changed at all, agents of the present invention can clearly be used in emulsifying systems without any problem.

## Claims

1. A film-forming agent for a cosmetic comprising a copolymer polymerized from a monomer composition containing (A) an unsaturated monomer having at least one polysiloxane structure in the molecule and (B) an unsaturated monomer having at least one acidic group or salt thereof in the molecule, wherein the content of monomer (A) in the monomer composition is at least 30 wt%.

2. A film-forming agent for a cosmetic according to claim 1, wherein the acid value of the copolymer is from 5 to 300 mgKOH/g.

3. A film-forming agent for a cosmetic according to claim 1, wherein the ratio of the weight of monomer (A) to the weight of monomer (B) (A/B) is from 1.0 to 20.0.

4. A film-forming agent for a cosmetic according to any of claims 1 to 3, wherein the unsaturated monomer having at least one polysiloxane structure in the molecule is selected from one represented by General Formula (1) 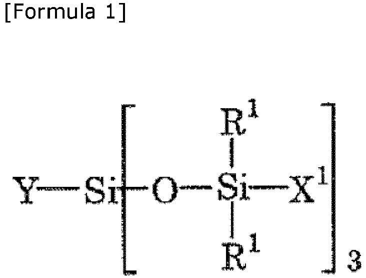 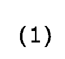 {In this formula, Y is a radically polymerizable organic group, R¹ is an alkyl or aryl group having from 1 to 10 carbon atoms, and X¹ is a silylalkyl group represented by the following formula where i = 1. 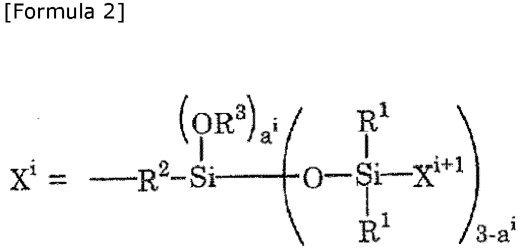 (In this formula, R¹ is the same as above, R² is an alkylene group having from 2 to 10 carbon atoms, R³ is an alkyl group having from 1 to 10 carbon atoms, Xⁱ⁺¹ is a hydrogen atom or a group selected from the group consisting of an alkyl group having from 1 to 10 carbon atoms, an aryl group, and a silylalkyl group mentioned above, i is an integer from 1 to 10 representing the number of levels of silylalkyl groups mentioned above, and aⁱ is an integer from 0 to 3.)}
or one represented by General Formula (2). 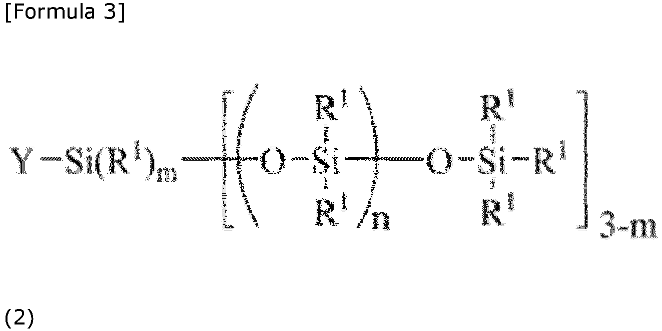 (In this formula, Y and R¹ are the same as above, m is 0, 1 or 2, and n is a number from 0 to 200 representing the average degree of polymerization.)

5. A film-forming agent for a cosmetic according to any of claims 1 to 4, wherein the monomer composition further comprises (C) a monomer having at least one carboxylic acid ester in the molecule.

6. A film-forming agent for a cosmetic according to any of claims 1 to 5, wherein monomer (B) is acrylic acid.

7. A film-forming agent for a cosmetic according to any of claims 1 to 6, further comprising at least one selected from the group consisting of (D) an oil and (E) an alcohol.

8. A film-forming agent for a cosmetic according to any of claims 1 to 7, further comprising (F) a surfactant.

9. A film-forming agent for a cosmetic according to any of claims 1 to 8, further comprising at least one selected from the group consisting of water, an inorganic powder, an organic powder, a colorant, a thickener, a gelling agent, an organically modified clay mineral, a silicone resin, a silicone gum, a silicone elastomer, an organically modified silicone, a UV protection component, a water-soluble polymer, an organic resin, a moisturizer, a preservative, an antioxidant, an antibacterial agent, a fragrance, a salt, a pH regulator, a chelating agent, an algefacient, an anti-inflammatory, a skin-beautifying component, a vitamin, an amino acid, a nucleic acid, a hormone, an inclusion compound, and an antistatic agent.

10. A cosmetic comprising a film-forming agent for a cosmetic according to any of claims 1 to 9.
